# EUROPEAN PATENT APPLICATION

(11) **EP 4 825 971 A1**
(43) Date of publication of application: **21.10.2026**
(21) Application number: 24903583.3
(22) Date of filing: 04.12.2024
(51) Int. Cl.: C12N 15/55, C11D 3/386, C12N 1/15, C12N 1/19, C12N 1/21, C12N 9/20, C12N 15/63

(54) **LIPASE VARIANT**

(30) Priority: 15.12.2023 JP 2023211838
(71) Applicant: Kao Corporation, Tokyo 103-8210 (JP)
(72) Inventor: NISHIGUCHI, Hiroki, Tokyo 103-8210 (JP); HIOKI, Takahiro, Tokyo 103-8210 (JP); WADA, Mayumi, Tokyo 103-8210 (JP); TERAI, Mika, Tokyo 103-8210 (JP); SHIBATA, Yumi, Tokyo 103-8210 (JP); KAWAHARA, Akihito, Tokyo 103-8210 (JP)
(74) Representative: Vossius & Partner Patentanwälte Rechtsanwälte mbB
(86) International application number: PCT/JP2024/042946
(87) International publication number: WO 2025/126926

(57) **Abstract**

Provided is a lipase variant which exhibits excellent stability. The lipase variant comprises one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase variant is a variant of a parent lipase and the parent lipase or the lipase variant has a sequence identity of at least 80% with the amino acid sequence of SEQ ID NO: 4 or 6.

## Description

### Field of the Invention

The present invention relates to a lipase variant.

### Background of the Invention

Lipases are useful in various uses such as a laundry detergent, a dishwashing detergent, oil processing, pulp treatment, feed, and pharmaceutical intermediate synthesis, and so on. In washing, lipases contribute to removal of stains including oil by hydrolyzing ester bonds in lipids to generate fatty acids.

A lipase for washing has been adopted for use in cleansing compositions for removing oil-based stains. Patent Literature 1 describes a method for washing oil stains by bringing a detergent including a sulfosuccinic acid alkyl ester together with a lipase into contact with oil stains and leaving it to stand without applying an external force. As described in Patent Literature 2, a cleansing composition for dishwashing is also used for washing stainless or resin sink areas, and a high concentration of a chelating agent such as citric acid may be blended therein in order to confer a function of cleaning limescale and the like attached to kitchen sink areas. A compositional feature of such a detergent is to contain a large amount of water compared to general detergents and to contain a solvent in an amount larger than that of a surfactant.

Patent Literature 1: JP-A-2021-17508
Patent Literature 2: JP-B-4776997

### Summary of the Invention

The present invention relates to the following 1) to 6).
1) A lipase variant, **comprising** one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase variant is a variant of a parent lipase and the parent lipase or the lipase variant has a sequence identity of at least 80% with the amino acid sequence of SEQ ID NO: 4 or 6.
2) A polynucleotide encoding the lipase variant according to 1).
3) A vector or DNA fragment comprising the polynucleotide according to 2).
4) A transformed cell comprising the vector or DNA fragment according to 3).
5) A cleansing composition comprising the lipase variant according to 1).
6) A method for improving stability of a lipase, comprising the step of substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase or a lipase after amino acid residue substitution of the lipase has a sequence identity of at least 80% with the amino acid sequence of SEQ ID NO: 4 or 6.

### Detailed Description of the Invention

All Patent Literatures, Non Patent Literatures, and other publications cited herein are incorporated by reference in their entireties.

As used herein, the term "lipase" refers to triacyl glycerol lipase (EC3.1.1.3) and means an enzyme group that has an activity which hydrolyzes an ester bond in a lipid to generate fatty acids. Lipase activity can be determined by measuring the rate of increase in absorbance due to release of 4-nitrophenol resulting from hydrolysis of 4-nitrophenyl butyrate. A specific procedure of the measurement of the lipase activity is described in detail in Examples mentioned later.

As used herein, the identity between amino acid sequences or nucleotide sequences is calculated by the Lipman-Pearson method (Science, 1985, 227: 1435-1441). Specifically, the identity is calculated by analysis using the homology analysis (Search homology) program of genetic information processing software GENETYX Ver. 12 by setting the Unit size to compare (ktup) to 2.

As used herein, the term "an identity of at least 80%" with respect to an amino acid sequence or nucleotide sequence refers to an identity of 80% or more, preferably 85% or more, more preferably 90% or more, further preferably 93% or more, further preferably 94% or more, further preferably 95% or more, further preferably 96% or more, further preferably 97% or more, further preferably 98% or more, further preferably 99% or more, and further preferably 99.5% or more.

As used herein, a "corresponding position" on an amino acid sequence or nucleotide sequence can be determined by aligning a target sequence and a reference sequence (for example, the amino acid sequence represented by SEQ ID NO: 4) so as to give the maximum homology. The alignment of amino acid sequences or nucleotide sequences can be carried out using a known algorithm, and the procedure thereof is known to those skilled in the art. For example, the alignment can be performed by using Clustal W multiple alignment program (Thompson, J.D., et al., 1994, Nucleic Acids Res., 22: 4673-4680) with the default set. Alternatively, Clustal W2 or Clustal omega, which are revised editions of Clustal W, can also be used. Clustal W, Clustal W2, and Clustal omega can be used on the website of, for example, the Clustal [www.clustal.org] operated by University College Dublin, the European Bioinformatics Institute: EBI [www.ebi.ac.uk/index.html], or the DNA data bank of Japan (DDBJ [www.ddbj.nig.ac.jp/searches-j.html]) operated by the National Institute of Genetics. A position in a target sequence aligned at any position in a reference sequence by the above-described alignment is considered to be a "position corresponding" to the position.

Those skilled in the art can further finely adjust and optimize the alignment of the amino acid sequence obtained above. The optimum alignment is preferably determined by considering the similarity of amino acid sequences, the frequency of gaps to be inserted, and so on. Here, the similarity of amino acid sequences refers to, when two amino acid sequences are aligned, the ratio (%) of the number of positions where the same or similar amino acid residues are present in both sequences to the number of the full-length amino acid residues. The similar amino acid residues mean that the amino acid residues have similar properties in the polarity or electric charge to each other and cause so-called conservative substitution among 20 amino acids constituting a protein. Groups consisting of such similar amino acid residues are well known to those skilled in the art, and examples thereof include, but not limited to, arginine and lysine or glutamine; glutamic acid and aspartic acid or glutamine; serine and threonine or alanine; glutamine and asparagine or arginine; and leucine and isoleucine.

As used herein, the term "amino acid residue" means 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

As used herein, an amino acid position and variant are designated using the accepted IUPAC one-letter amino acid abbreviation as follows.

An amino acid at a predetermined position is designated by [amino acid, position]. For example, valine at position 220 is shown as "V220".

"Substitution" of an amino acid is designated by [original amino acid, position, substituted amino acid]. For example, substitution of valine at position 220 with histidine is designated as "V220H".

A variant including a plurality of modifications is indicated by an addition symbol ("+"). For example, "V220H + N272G" represents substitution of valine at position 220 with histidine and substitution of asparagine at position 272 with glycine.

If different modifications may be introduced at one position, the different modifications are separated by slashes ("/"). For example, "G22C/M" represents substitution of glycine at position 22 with cysteine or methionine.

As used herein, the term "operable linkage" between a regulatory region, such as a promoter, and a gene indicates that a gene and a regulatory region are linked such that the gene can be expressed under the control of the regulatory region. The procedure of "operable linkage" between a gene and a regulatory region is known to those skilled in the art.

As used herein, the terms "upstream" and "downstream" with respect to a gene refer to the upstream and downstream of the gene in the transcription direction. For example, "a gene placed downstream of a promoter" means that the gene is present on the 3' side of the promoter in a DNA sense strand, and upstream of a gene means a region on the 5' side of the gene in a DNA sense strand.

Although the stability of a lipase blended in a cleansing composition which contains a large amount of water compared to general detergents and contains a solvent in an amount larger than that of a surfactant had been unknown in such severe environments for enzymes, the present inventors revealed as a result of studies that the stability is significantly reduced compared to the case where a lipase is blended in general detergents. Accordingly, the present invention relates to providing lipase which exhibits excellent stability.

The present inventors obtained a lipase variant having improved stability in the presence of a large amount of water and/or a larger amount of a solvent than that of a surfactant compared to a parent lipase.

The lipase variant of the present invention has improved stability compared to the parent lipase. This lipase variant can be suitably blended even in a cleansing composition containing a large amount of water and/or a larger amount of a solvent than that of a surfactant.

### <1. Lipase variant>

The lipase variant of the present invention is a variant of a parent lipase, comprising one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the parent lipase or the lipase variant has a sequence identity of at least 80% with the amino acid sequence of SEQ ID NO: 4 or 6.

Specifically, the "variant" means a polypeptide that includes one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 in the parent lipase and has lipase activity. Such substitution of the amino acid residue at a predetermined position is a modification for improving stability. Thus, the variant has improved stability compared to the parent lipase. Here, the stability more specifically refers to stability in the presence of a large amount of water and/or a larger amount of a solvent than that of a surfactant.

The "parent lipase" means a reference lipase in which a modification is to be made in order to bring about the variant of the present invention and in the present invention, is a lipase having a sequence identity of at least 80% with the amino acid sequence of SEQ ID NO: 4 or 6. The parent lipase may be a natural (wild-type) polypeptide or its variant.

An example of the parent lipase includes a polypeptide which consists of an amino acid sequence having an identity of at least 80% with the amino acid sequence of SEQ ID NO: 4 and has lipase activity. Here, the lipase consisting of the amino acid sequence of SEQ ID NO: 4 is a lipase which has amino acid residue substitutions I44T, L120S, S130A, A134T, A135V, L136M, and S137N in an amino acid sequence constituting lipase CnLip (NCBI Accession No. WP_061278013.1) derived from *Cedecea neteri* (CnLipA having excellent detergency described in Japanese Patent Application No. 2023-025141) and further has amino acid residue substitutions F16L, I47L, A91V, E97D, and A149E, and is CnLipAm shown in Examples described later. Both the CnLip and the CnLipA are capable of serving as the parent lipase of the variant of the present invention.

Another example of the parent lipase includes a polypeptide which consists of an amino acid sequence having an identity of at least 80% with the amino acid sequence of SEQ ID NO: 6 and has lipase activity. Here, the lipase consisting of the amino acid sequence of SEQ ID NO: 6 is a lipase which has amino acid residue substitutions I45T, L121S, S131A, A135T, A136V, and S138N in an amino acid sequence constituting lipase CspLip (NCBI Accession No. WP_016537805.1) derived from *Cedecea* sp. (CspLipA excellent in detergency described in Japanese Patent Application No. 2023-025141), and is CspLipA shown in Examples described later.

The "substitution" of the amino acid residue at a predetermined position means replacement of an amino acid at a position with a different amino acid.

In the present invention, the position of amino acid residue substitution may be two or more positions and is preferably two or more positions, more preferably from 2 to 20 positions, more preferably from 3 to 10 positions, from the viewpoint of stability.

The variant is preferably a lipase having an identity of at least 80% with the amino acid sequence of SEQ ID NO: 4 or 6 from the viewpoint of stability and detergency.

The variant may include any number of conservative amino acid substitution as long as the variant retains the properties described above.

A suitable mode of each of the substitutions of the amino acid residues at positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286, numbered according to SEQ ID NO: 4 in the lipase variant of the present invention will be shown below.
Specifically, L16 is preferably substituted with F (L16F) ;
G22 is preferably substituted with C or M (G22C/M);
Y29 is preferably substituted with F (Y29F);
Y43 is preferably substituted with W (Y43W);
A45 is preferably substituted with V (A45V);
T46 is preferably substituted with Q (T46Q);
L50 is preferably substituted with **I,** M, or V (L50I/M/V) ;
S120 is preferably substituted with A or Q (S120A/Q) ;
K123 is preferably substituted with I or T (K123I/T);
R126 is preferably substituted with E (R126E);
T134 is preferably substituted with A (T134A);
L142 is preferably substituted with F (L142F);
A145 is preferably substituted with C or Q (A145C/Q);
G156 is preferably substituted with A (G156A);
K174 is preferably substituted with D (K174D);
A181 is preferably substituted with K (A181K);
Q186 is preferably substituted with E (Q186E);
K188 is preferably substituted with Q (K188Q);
L190 is preferably substituted with F (L190F);
D191 is preferably substituted with A, E, or V (D191A/E/V) ;
L208 is preferably substituted with C (L208C);
H209 is preferably substituted with K (H209K);
M212 is preferably substituted with L (M212L);
L215 is preferably substituted with C (L215C);
V220 is preferably substituted with H (V220H);
A221 is preferably substituted with H (A221H);
L223 is preferably substituted with A, E, S, or V (L223A/E/S/V) ;
A224 is preferably substituted with F, Q, or Y (A224F/Q/Y) ;
F225 is preferably substituted with T (F225T);
L228 is preferably substituted with W (L228W);
R233 is preferably substituted with K, Q, or T (R233K/Q/T);
F234 is preferably substituted with W (F234W);
S256 is preferably substituted with N (S256N);
V260 is preferably substituted with A, C, F, G, L, M, or T (V260A/C/F/G/L/M/T);
L266 is preferably substituted with P (L266P);
V269 is preferably substituted with I, L, or M (V269I/L/M) ;
N272 is preferably substituted with C, G, or T (N272C/G/T);
T274 is preferably substituted with I or Q (T274I/Q) ;
K278 is preferably substituted with A (K278A); and
R286 is preferably substituted with M (R286M).

Next, a suitable combination of mutations that contributes to improved stability will be shown in the following (A) to (D). Thus, a variant having at least any combination of mutations of the following (A) to (D) is a variant which contributes to improved stability:
(A) N272C/G/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, T274I/Q, K278A, and R286M numbered according to SEQ ID NO: 4,
(B) V220H substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, A221H, L223A/E/S/V, A224F/Q/Y, F225T, L228W, R233K/Q/T, F234W, S256N, V260A/C/F/G/L/M/T, L266P, V269I/L/M, N272C/G/T, T274I/Q, K278A, and R286M numbered according to SEQ ID NO: 4,
(C) V260A/C/F/G/L/M/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, L266P, V269I/L/M, N272C/G/T, T274I/Q, K278A, and R286M numbered according to SEQ ID NO: 4, and
(D) L208C substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T274I/Q, K278A, and R286M numbered according to SEQ ID NO: 4.

A more suitable combination of mutations that contributes to improved stability will be shown in the following (A') to (D'). Thus, a variant having at least any combination of mutations of the following (A') to (D') is a variant which more contributes to improved stability:
(A') N272C/G substitution and one or more substitutions selected from the group consisting of L16F, G22M, Y29F, Y43W, L50I/V, S120A/Q, K123I/T, R126E, L142F, G156A, A181K, Q186E, K188Q, L190F, D191V, L208C, M212L, V220H, A221H, L223V, A224Q, F225T, L228W, R233K/T, F234W, S256N, V260A/C/L, V269I/L, T274Q, and K278A numbered according to SEQ ID NO: 4,
(B') V220H substitution and one or more substitutions selected from the group consisting of L16F, G22C/M, Y43W, L50I/V, S120Q, K123I/T, R126E, L142F, G156A, A181K, Q186E, K188Q, L190F, D191A/E/V, L208C, M212L, A221H, L223V, A224Q, F225T, L228W, R233K/Q, F234W, S256N, V260A/C/L, V269I/L/M, N272C/G, T274Q, and K278A numbered according to SEQ ID NO: 4,
(C') V260A/C/L substitution and one or more substitutions selected from the group consisting of L16F, G22M, T46Q, L50I/V, K123I, R126E, L142F, G156A, L190F, D191A/E, L208C, M212L, V220H, A224F/Q/Y, F225T, L228W, R233K/Q, R234W, V269I/M, N272C/G, and K278A numbered according to SEQ ID NO: 4, and
(D') L208C substitution and one or more substitutions selected from the group consisting of L16F, G22C, L50I/V, K123I, R126E, K188Q, L190F, D191A, M212L, V220H, L223V, A224Q/Y, F225T, L228W, R233K, F234W, S256N, V260A/C/L, V269I, N272C/G, K278A, and R286M numbered according to SEQ ID NO: 4.

Among the combinations of mutations of (A') to (D'), the following combinations of mutations are preferred from the viewpoint of stability:
(A') N272C substitution and one or more substitutions selected from the group consisting of L16F, G22M, Y29F, Y43W, L50I/V, S120A/Q, K123I, R126E, L142F, G156A, A181K, K188Q, D191V, L208C, V220H, A221H, L223V, A224Q, F225T, R233K/T, F234W, S256N, V260A/C/L, V269I, and T274Q numbered according to SEQ ID NO: 4,
(B') V220H substitution and one or more substitutions selected from the group consisting of G22C/M, L50I/V, K123I/T, R126E, L142F, G156A, A181K, Q186E, D191V, L208C, M212L, A221H, L223V, L228W, R233K/Q, F234W, S256N, V260C/L, V269I/L/M, N272C/G, T274Q, and K278A numbered according to SEQ ID NO: 4,
(C') V260C substitution and one or more substitutions selected from the group consisting of L50I/V and L142F numbered according to SEQ ID NO: 4, or (C') V260L substitution and one or more substitutions selected from the group consisting of G22M, L50I/V, G156A, D191A/E, L208C, M212L, V220H, A224F/Q/Y, L228W, V269I/M, and N272G numbered according to SEQ ID NO: 4, and
(D') L208C substitution and one or more substitutions selected from the group consisting of L16F, G22C, R126E, K188Q, D191A, V220H, L223V, A224Q/Y, L228W, S256N, V260A/C/L, V269I, N272C/G, K278A, and R286M numbered according to SEQ ID NO: 4.

Among the combinations of mutations of (A') to (D'), the following combinations of mutations are more preferred from the viewpoint of stability:
(A') N272C substitution and one or more substitutions selected from the group consisting of G22M, K123I, G156A, K188Q, L208C, V220H, A221H, L223V, A224Q, F225T, R233T, S256N, V260A/C/L, and V269I numbered according to SEQ ID NO: 4,
(B') V220H substitution and one or more substitutions selected from the group consisting of G22C/M, G156A, L208C, M212L, L228W, S256N, V260C/L, V269I/L/M, and N272C/G numbered according to SEQ ID NO: 4,
(C') V260C substitution and one or more substitutions selected from the group consisting of L50I/V and L142F numbered according to SEQ ID NO: 4, or V260L substitution and one or more substitutions selected from the group consisting of G22M, G156A, L208C, V220H, L228W, V269I/M, and N272G numbered according to SEQ ID NO: 4, and
(D') L208C substitution and one or more substitutions selected from the group consisting of L16F, G22C, K188Q, V220H, L228W, S256N, V260A/C/L, V269I, and N272C/G numbered according to SEQ ID NO: 4.

A further suitable combination of mutations that contributes to improved stability is V220H substitution and N272C/G substitution; V260L substitution and V269I substitution; L208C substitution, V220H substitution, and N272C substitution; L142F substitution, V220H substitution, and N272G substitution; V260L substitution, V269I substitution, and N272G; L208C substitution, V220H substitution, V269I substitution, and N272C substitution; L50V substitution, L142F substitution, V220H substitution, and N272G substitution; L50V substitution, V260L substitution, V269I substitution, and N272G substitution; L208C substitution, V220H substitution, S256N substitution, V269I substitution, and N272C substitution; or L50V substitution, L142F substitution, V220H substitution, V260L substitution, and N272G substitution numbered according to SEQ ID NO: 4. Thus, a variant having any of these combinations of mutations is a variant which further contributes to improved stability.

A further suitable mutation or combination of mutations in the lipase variant of the present invention is shown in the following Table 1 (Tables 1-1 to 1-5) and Table 2 (Tables 2-1 to 2-3). Thus, a variant having any of the following mutations or combinations of mutations is a variant which particularly contributes to improved stability.

**[Table 1]**

| Table 1-1 | Table 1-2 | Table 1-3 | Table 1-4 | Table 1-5 |
|---|---|---|---|---|
| L16F | L223A | N272C+L16F | V220H+K123T | V260L+D191A |
| G22C | L223E | N272C+G22M | V220H+R126E | V260L+D191E |
| G22M | L223S | N272C+Y29F | V220H+L142F | V260L+L208C |
| Y29F | L223V | N272C+Y43W | V220H+G156A | V260L+M212L |
| Y43W | A224F | N272C+L501 | V220H+A181K | V260L+A224F |
| A45V | A224Q | N272C+L50V | V220H+Q186E | V260L+A224Q |
| T46Q | A224Y | N272C+S120A | V220H+K188Q | V260L+A224Y |
| L501 | F225T | N272C+S120Q | V220H+L190F | V260L+L228W |
| L50M | L228W | N272C+K1231 | V220H+D191A | V260L+R233K |
| L50V | R233K | N272C+K123T | V220H+D191E | V260L+R233Q |
| S120A | R233Q | N272C+R126E | V220H+D191V | V260L+F234W |
| S120Q | R233T | N272C+L142F | V220H+L208C | V260L+V2691 |
| K1231 | F234W | N272C+G156A | V220H+M212L | V260L+V269M |
| K123T | V260A | N272C+A181K | V220H+A221H | V260L+N272G |
| R126E | V260C | N272C+Q186E | V220H+L223V | L208C+L16F |
| T134A | V260F | N272C+K188Q | V220H+L228W | L208C+G22C |
| L142F | V260G | N272C+D191V | V220H+R233K | L208C+L501 |
| A145C | V260L | N272C+L208C | V220H+R233Q | L208C+R126E |
| A145Q | V260M | N272C+M212L | V220H+F234W | L208C+K188Q |
| K174D | V260T | N272C+V220H | V220H+S256N | L208C+D191A |
| A181K | L266P | N272C+A221H | V220H+V260C | L208C+L223V |
| K188Q | V2691 | N272C+L223V | V220H+V260L | L208C+A224Q |
| L190F | V269L | N272C+A224Q | V220H+V2691 | L208C+A224Y |
| D191A | V269M | N272C+F225T | V220H+V269L | L208C+L228W |
| D191E | N272C | N272C+R233K | V220H+V269M | L208C+S256N |
| D191V | N272G | N272C+R233T | V220H+N272G | L208C+V260A |
| L208C | N272T | N272C+F234W | V220H+T274Q | L208C+V260C |
| H209K | T2741 | N272C+S256N | V220H+K278A | L208C+V260L |
| M212L | T274Q | N272C+V260A | V260C+L16F | L208C+V2691 |
| L215C | K278A | N272C+V260C | V260C+L501 | L208C+N272G |
| V220H | R286M | N272C+V260L | V260C+L50V | L208C+K278A |
| A221H | | N272C+V2691 | V260C+L142F | L208C+R286M |
| | | N272C+T274Q | V260C+A224Q | |
| | | V220H+G22C | V260C+N272G | |
| | | V220H+G22M | V260L+L16F | |
| | | V220H+Y43W | V260L+G22M | |
| | | V220H+L501 | V260L+T46Q | |
| | | V220H+L50V | V260L+L50I | |
| | | V220H+S120Q | V260L+L50V | |
| | | V220H+K1231 | V260L+G156A | |

**[Table 2]**

| Table 2-1 | Table 2-2 | Table 2-3 |
|---|---|---|
| V220H+N272C+L16F | V220H+N272C+L208C+L16F | V220H+N272G+L142F+L50V+L16F |
| V220H+N272C+L50V | V220H+N272C+L208C+L50V | V220H+N272G+L142F+L50V+K123I |
| V220H+N272C+K1231 | V220H+N272C+L208C+K1231 | V220H+N272G+L142F+L50V+V260L |
| V220H+N272C+R126E | V220H+N272C+L208C+V260L | V220H+N272C+L208C+V269I+L16F |
| V220H+N272C+L142F | V220H+N272C+L208C+V2691 | V220H+N272C+L208C+V2691+L50V |
| V220H+N272C+G156A | V220H+N272G+L142F+L50V | V220H+N272C+L208C+V2691+S256N |
| V220H+N272C+L190F | V220H+N272G+L142F+L16F | V220H+N272C+L208C+V2691+V260L |
| V220H+N272C+L208C | V220H+N272G+L142F+L50V | V220H+N272C+L208C+S256N+R126E |
| V220H+N272C+M212L | V220H+N272G+L142F+K123I | V260L+V2691+N272G+L50V+L501 |
| V220H+N272C+L228W | V220H+N272G+L142F+A224Q | V260L+V269I+N272G+L50V+R126E |
| V220H+N272C+R233K | V220H+N272G+L142F+V260L | V260L+V269I+N272G+L50V+L208C |
| V220H+N272C+F234W | V260L+V2691+N272G+L501 | V260L+V2691+N272G+L50V+A224Q |
| V220H+N272C+V260A | V260L+V2691+N272G+L50V | V220H+N272G+L142F+L50V+V260L+L16F |
| V220H+N272C+V260L | V260L+V2691+N272G+L208C | V220H+N272G+L142F+L50V+V260L+K123I |
| V220H+N272C+V2691 | V260L+V2691+N272G+A224Q | V220H+N272G+L142F+L50V+V260L+R126E |
| V220H+N272C+V269L | | V220H+N272C+L208C+V269I+S256N+L50V |
| V220H+N272C+K278A | | V220H+N272C+L208C+V2691+S256N+R126E |
| V220H +N272G+L16F | | V260L+V2691+N272G+L50V+A224Q+M212L |
| V220H +N272G+L501 | | V260L+V2691+N272G+L50V+A224Q+K278A |
| V220H +N272G+L50V | | V220H+N272G+L142F+L50V+V260L+L16F+M212L |
| V220H +N272G+K1231 | | V220H+N272G+L142F+L50V+V260L+L16F+F225T |
| V220H +N272G+R126E | | V220H+N272G+L142F+L50V+V260L+L16F+R233K |
| V220H +N272G+L142F | | V220H+N272G+L142F+L50V+V260L+L16F+K278A |
| V220H +N272G+L208C | | V260L+V2691+N272G+L50V+A224Q+K278A+M212L |
| V220H +N272G+A224Q | | V220H+N272C+L208C+V269I+S256N+R126E+F225T |
| C220H+N272G+F225T | | V220H+N272C+L208C+V269I+S256N+R126E+F234W |
| C220H+N272G+R233K | | V220H+N272C+L208C+V2691+S256N+R126E+K278A |
| V220H +N272G+V260L | | V220H+N272G+L142F+L50V+V260L+L16F+F225T+K278A |
| V260L+V2691+L501 | | V220H+N272C+L208C+V2691+S256N+R126E+K278A+L190F |
| V260L+V2691+L50V | | V220H+N272C+L208C+V2691+S256N+R126E+K278A+M212L |
| V260L+V269I+L190F | | V220H+N272C+L208C+V2691+S256N+R126E+K278A+L223V |
| V260L+V2691+L208C | | V220H+N272C+L208C+V2691+S256N+R126E+K278A+F225T |
| V260L+V2691+M212L | | V220H+N272C+L208C+V2691+S256N+R126E+K278A+R233K |
| V260L+V2691+A224Q | | V220H+N272C+L208C+V2691+S256N+R126E+K278A+M212L+L190F |
| V260L+V2691+R233K | | V220H+N272C+L208C+V2691+S256N+R126E+K278A+M212L+L223V |
| V260L+V2691+N272G | | V220H+N272C+L208C+V269I+S256N+R126E+K278A+M212L+F225T |
| V260L+V2691+K278A | | V220H+N272C+L208C+V269I+S256N+R126E+K278A+M212L+L228W |
| | | V220H+N272C+L208C+V269I+S256N+R126E+K278A+M212L+R233K |
| | | V220H+N272C+L208C+V269I+S256N+R126E+K278A+M212L+F234W |

Positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 as described above correspond to positions L17, G23, Y30, Y44, A46, T47, L51, S121, T124, R127, T135, L143, A146, G157, K175, A182, E187, K189, L191, V192, L209, H210, A213, L216, V221, A222, L224, A225, F226, L229, R234, F235, S257, V261, L267, V270, N273, T275, Q279, and R287, respectively, numbered according to SEQ ID NO: 6. Accordingly, when the parent lipase has a sequence identity of at least 80% with the amino acid sequence of SEQ ID NO: 6, each of the substitutions of the amino acid residues at positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 can be interpreted as the corresponding substitutions of the amino acid residues at positions corresponding to positions L17, G23, Y30, Y44, A46, T47, L51, S121, T124, R127, T135, L143, A146, G157, K175, A182, E187, K189, L191, V192, L209, H210, A213, L216, V221, A222, L224, A225, F226, L229, R234, F235, S257, V261, L267, V270, N273, T275, Q279, and R287 numbered according to SEQ ID NO: 6, and the amino acid residues after substitution are preferably those mentioned above. However, when the parent lipase has the amino acid sequence of SEQ ID NO: 6, substitution at position 124 with T, substitution at position 187 with E, and substitution at position 192 with V are preferably not included. When the parent lipase has a sequence identity of at least 80% with the amino acid sequence of SEQ ID NO: 4, the parent lipase preferably has, as the amino acid residues at the positions substituted in the variant of the present invention, corresponding amino acid residues among L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, and more preferably has amino acid residues L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4. When the parent lipase has a sequence identity of at least 80% with the amino acid sequence of SEQ ID NO: 6, the parent lipase preferably has, as the amino acid residues at the substitution positions in the variant of the present invention, corresponding amino acid residues among L17, G23, Y30, Y44, A46, T47, L51, S121, T124, R127, T135, L143, A146, G157, K175, A182, E187, K189, L191, V192, L209, H210, A213, L216, V221, A222, L224, A225, F226, L229, R234, F235, S257, V261, L267, V270, N273, T275, Q279, and R287 numbered according to SEQ ID NO: 6, and more preferably has amino acid residues L17, G23, Y30, Y44, A46, T47, L51, S121, T124, R127, T135, L143, A146, G157, K175, A182, E187, K189, L191, V192, L209, H210, A213, L216, V221, A222, L224, A225, F226, L229, R234, F235, S257, V261, L267, V270, N273, T275, Q279, and R287 numbered according to SEQ ID NO: 6.

The lipase variant of the present invention can be produced by substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 in the parent lipase. Here, the parent lipase or the lipase variant has a sequence identity of at least 80% with the amino acid sequence of SEQ ID NO: 4 or 6.

### <2. Polynucleotide encoding lipase variant of the present invention>

The lipase variant of the present invention can be produced using various mutagenesis techniques known in the art. For example, the lipase variant can be produced by mutating a polynucleotide encoding the amino acid residue to be substituted in a parent lipase gene (reference lipase gene) encoding the reference amino acid sequence into a polynucleotide encoding an amino acid residue after substitution, and further expressing the variant from the variant gene.

In the present invention, as the method for mutating an amino acid residue of a parent lipase, various mutagenesis techniques known in the art can be used. For example, a polynucleotide encoding the lipase variant of the present invention can be obtained by mutating a nucleotide sequence encoding an amino acid residue to be mutated into a nucleotide sequence encoding the amino acid residue after mutation in a polynucleotide encoding the amino acid sequence of a parent lipase (hereinafter, also referred to as a parent gene).

Basically, introduction of a desired mutation into a parent gene can be performed using various site-directed mutagenesis methods known to those skilled in the art. The site-directed mutagenesis can be performed by an arbitrary method, such as an inverse PCR method or an annealing method. Commercially available site-directed mutagenesis kits (for example, QuickChange II Site-Directed Mutagenesis Kit and QuickChange Multi Site-Directed Mutagenesis Kit of Stratagene) can also be used.

Site-directed mutagenesis into a parent gene can be most generally performed using a mutation primer including a nucleotide mutation to be introduced. The mutation primer may be designed so as to anneal with a region including a nucleotide sequence that encodes an amino acid residue to be mutated in a parent gene and to include a nucleotide sequence having a nucleotide sequence (codon) that encodes an amino acid residue after the mutation instead of the nucleotide sequence (codon) encoding the amino acid residue to be mutated. Nucleotide sequences (codons) encoding amino acid residues before and after mutation can be appropriately recognized and selected by those skilled in the art based on ordinary textbooks and the like. Alternatively, as site-directed mutagenesis, a method of linking DNA fragments into one by SOE (splicing by overlap extension)-PCR (Gene, 1989, 77(1): 61-68), wherein the DNA fragments are separately obtained by amplifying both the upstream and downstream sides of a mutation site with two complementary primers including the nucleotide mutation to be introduced can be used.

A template DNA including a parent gene can be prepared from a microorganism that produces the above-described parent lipase by extracting genomic DNA by a usual method or extracting RNA and synthesizing cDNA therefrom by reverse transcription. Alternatively, a corresponding nucleotide sequence chemically synthesized based on the amino acid sequence of a parent lipase may be used as a template DNA. DNA sequences including nucleotide sequences encoding lipases consisting of amino acid sequences of SEQ ID NOs: 4 and 6 are represented by SEQ ID NOs: 3 and 5, respectively.

A mutation primer can be produced by a known oligonucleotide synthesis method such as a phosphoramidite method (Nucleic Acids Research, 1989, 17: 7059-7071). Such primer synthesis can also be carried out using, for example, a commercially available oligonucleotide synthesis apparatus (for example, manufactured by ABI Inc.). A polynucleotide encoding a lipase variant with a desired mutation of the present invention can be obtained using a primer set including the mutation primer and performing site-directed mutagenesis as described above using a parent gene as a template DNA.

The polynucleotide encoding the lipase variant of the present invention can include a single-stranded or double-stranded DNA, cDNA, RNA, or another artificial nucleic acid. The DNA, cDNA, and RNA may be chemically synthesized. The polynucleotide may include a nucleotide sequence of an untranslated region (UTR), in addition to an open reading frame (ORF). The polynucleotide may be codon-optimized in accordance with the type of a transformant for producing the variant polypeptide of the present invention. Information on codons to be used by various organisms is available from the Codon Usage Database ([www.kazusa.or.jp/codon/]).

### <3. Vector or DNA fragment>

The obtained polynucleotide encoding the lipase variant of the present invention can be incorporated into a vector. The type of the vector containing the polynucleotide is not particularly limited and may be any vector such as a plasmid, phage, phagemid, cosmid, virus, YAC vector, and shuttle vector. The vector is, but not limited to, preferably, a vector capable of being amplified in bacteria, preferably, in *Bacillus* bacteria (for example, *Bacillus subtilis* or its mutant strain), more preferably, an expression vector capable of inducing expression of a transgene in *Bacillus* bacteria. In particular, a shuttle vector, which is a vector capable of replicating in both *Bacillus* bacteria and other organisms, can be suitably used for recombinant production of the lipase variant of the present invention. Preferable examples of the vector include, but not limited to, shuttle vectors, such as pHA3040SP64, pHSP64R, and pASP64 (Japanese Patent No. 3492935), pHY300PLK (expression vector capable of transforming both *Escherichia coli* and *Bacillus subtilis;* Jpn J. Genet., 1985, 60: 235-243), and pAC3 (Nucleic Acids Res., 1988, 16: 8732); and plasmid vectors that can be used for transformation of *Bacillus* bacteria, such as pUB110 (J. Bacteriol., 1978, 134: 318-329) and pTA10607 (Plasmid, 1987, 18: 8-15). In addition, plasmid vectors derived from *Escherichia coli* (e.g., pET22b(+), pBR322, pBR325, pUC57, pUC118, pUC119, pUC18, pUC19, and pBluescript) can also be used.

The above vectors can include a DNA replication initiation region or a DNA region including replication origin. Alternatively, in the above vectors, a control sequence may be operably linked to the upstream of a polynucleotide encoding the lipase variant of the present invention (i.e., lipase variant gene), wherein the control sequence is a promoter region for initiating transcription of the gene, terminator region, secretory signal region for extracellularly secreting the expressed protein, or the like.

The type of the control sequence, such as the promoter region, terminator region, and secretory signal region, is not particularly limited, and a promoter or secretory signal sequence that is usually used can be appropriately selected and used depending on the host into which the sequence is introduced. For example, suitable examples of the control sequence capable of being incorporated into a vector include a promoter of the cellulase gene of *Bacillus* sp. KSM-S237 strain and a secretory signal sequence.

Alternatively, a marker gene (e.g., a gene resistant to drugs, such as ampicillin, neomycin, kanamycin, and chloramphenicol) for selecting a host into which the vector has been appropriately introduced may be further incorporated into the vector of the present invention. Alternatively, when an auxotrophic strain is used as the host, a gene encoding an enzyme that synthesizes a required nutrient may be incorporated into a vector as a marker gene. Alternatively, when a selection medium that requires a specific metabolism for growth is used, a gene related to the metabolism may be incorporated into a vector as a marker gene. Examples of such a metabolism-related gene include an acetamidase gene for using acetamide as a nitrogen source.

The polynucleotide encoding the lipase variant of the present invention may be linked to a control sequence and a marker gene by a method known in the art such as an SOE (splicing by overlap extension)-PCR method (Gene, 1989, 77: 61-68). The procedure of introducing the linked fragments into a vector is well known in the art.

### <4. Transformed cell>

A transformed cell of the present invention can be obtained by introducing a vector including a polynucleotide encoding the lipase variant of the present invention into a host or by introducing a DNA fragment including a polynucleotide encoding the lipase variant of the present invention into the genome of a host.

Examples of the host cell include microorganisms such as bacteria and fungi. Examples of the bacteria include *Escherichia coli* and bacteria belonging to *Staphylococcus, Enterococcus, Listeria,* and *Bacillus,* and among them, preferred are *Escherichia coli* and *Bacillus* bacteria, more preferred are *Bacillus* bacteria, and further preferred are *Bacillus subtilis* (e.g., *Bacillus subtilis* Marburg No. 168 strain and its mutant strain). Examples of the *Bacillus subtilis* mutant strain include a nine protease-deficient strain KA8AX described in J. Biosci. Bioeng., 2007, 104(2): 135-143 and D8PA strain that is an eight protease-deficient strain with improved protein folding efficiency described in Biotechnol. Lett., 2011, 33(9): 1847-1852. Examples of the fungus include *Trichoderma, Aspergillus,* and *Rhizopus.*

As the method for introducing a vector into a host, a method that is usually used in the art, such as a protoplast method and an electroporation method, can be used. A desired transformant into which a vector has been introduced can be obtained by selecting a strain in which the introduction has been appropriately performed by using expression of a marker gene, auxotrophy, or the like as an indicator.

Alternatively, a fragment in which a polynucleotide encoding the lipase variant of the present invention, a control sequence, and a marker gene are linked can also be directly introduced into the genome of a host. For example, a DNA fragment in which a sequence complementary to the genome of a host has been added to both ends of the linked fragment is constructed by an SOE-PCR method or the like and is introduced into a host to cause homologous recombination between the host genome and the DNA fragment. Consequently, the polynucleotide encoding the lipase variant of the present invention is introduced into the genome of the host.

When the thus-obtained transformant into which a polynucleotide encoding the lipase variant of the present invention or a vector including it has been introduced is cultured in an appropriate medium, a gene encoding the protein on the vector is expressed to generate the lipase variant of the present invention. The medium that is used for culturing the transformant can be appropriately selected depending on the type of the microorganism of the transformant by those skilled in the art.

Alternatively, the lipase variant of the present invention may be expressed from a polynucleotide encoding the lipase variant of the present invention or its transcriptional product using a cell-free translation system. The "cell-free translation system" is an in vitro transcription translation system or in vitro translation system constructed by adding a reagent, such as amino acid, necessary for translation of a protein to a suspension obtained by mechanically lysing the cells as the host.

The lipase variant of the present invention generated by the culture or cell-free translation system can be isolated or purified by using general methods that are used in protein purification, such as centrifugation, ammonium sulfate precipitation, gel chromatography, ion exchange chromatography, and affinity chromatography, alone or in appropriate combination. The protein collected from the culture may be further purified by a known method.

### <5. Method for improving stability of lipase>

The thus-obtained lipase variant of the present invention has improved stability, more specifically, improved stability in the presence of a large amount of water and/or a larger amount of a solvent than that of a surfactant, compared to the parent lipase.

The "stability" means an ability to maintain lipase activity. The "improved stability" means an improved ability to maintain lipase activity compared to the parent lipase. The "improved stability in the presence of a large amount of water and/or a larger amount of a solvent than that of a surfactant" means an improved ability to maintain lipase activity in the presence of a large amount of water and/or a larger amount of a solvent than that of a surfactant compared to the parent lipase.

Here, the stability in the presence of a large amount of water means an ability to maintain lipase activity when the lipase is preserved in a cleansing composition having a water content of from 60 to 99 mass%. The stability in the presence of a larger amount of a solvent than that of a surfactant means an ability to maintain lipase activity in a cleansing composition having a larger content of a solvent than that of a surfactant or in a washing solution having a larger content of a solvent than that of a surfactant, prepared by dissolving or diluting the cleansing composition in or with water.

Examples of the surfactant and the solvent include surfactants and solvents mentioned later.

The stability of the lipase can be evaluated using a method well known in the art. The stability of the lipase can be evaluated, for example, by adding the lipase to an appropriate cleansing composition or a washing solution prepared by dissolving or diluting the cleansing composition in or with water, measuring lipase activity before and after treatment for a predetermined time, and calculating a half-life of the activity on the basis of a deactivation rate per unit time by the treatment with the activity value of the sample before the treatment as initial activity. A value obtained by dividing the half-life of the lipase variant by the half-life of the parent lipase indicates relative stability of the lipase variant. When this value exceeds 1, the lipase variant is evaluated as having improved stability compared to the parent lipase. Examples of the washing solution include, but not limited to, a model washing solution having the composition shown in Table 3 in Examples described later.

The stability of the lipase variant of the present invention can be preferably 1.05 or more, more preferably 1.1 or more, further more preferably 1.2 or more, as relative stability after treatment at a given temperature for a given time (e.g., at 45°C for 1 hour).

The present invention also provides a method for improving stability of a lipase. The method for improving stability of a lipase according to the present invention is a method comprising the step of substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 in the lipase, wherein the lipase or a lipase after amino acid residue substitution of the lipase has a sequence identity of at least 80% with the amino acid sequence of SEQ ID NO: 4 or 6. The detailed substitution of the amino acid residue is the same as in the case of production of the lipase variant mentioned above.

### <6. Cleansing composition>

The lipase variant of the present invention is useful as an enzyme that is blended in various cleansing compositions and is particularly useful as an enzyme that is blended in a cleansing composition suitable for low-temperature washing.

Here, the "low temperature" is, for example, 40°C or less, 35°C or less, 30°C or less, or 25°C or less and is, for example, 5°C or more, 10°C or more, or 15°C or more and is, for example, from 5°C to 40°C, from 10°C to 35°C, from 15°C to 30°C, or from 15°C to 25°C.

The amount of the lipase variant of the present invention blended in the cleansing composition is not specifically limited as long as the lipase variant exhibits the activity and is, based on 1 kg of the cleansing composition, for example, preferably 0.1 mg or more, more preferably 1 mg or more, and further preferably 5 mg or more and is preferably 5000 mg or less, further preferably 1 000 mg or less, and further preferably 500 mg or less, and is preferably from 0.1 to 5 000 mg, more preferably from 1 to 1 000 mg, and further preferably from 5 to 500 mg.

The cleansing composition preferably includes a surfactant sulfosuccinic acid ester or a salt thereof, in addition to the lipase variant of the present invention. The sulfosuccinic acid ester or a salt thereof is known as a component that is blended in cleansing compositions (for example, JP-A-2019-182911). The sulfosuccinic acid ester or a salt thereof is preferably a sulfosuccinic acid branched alkyl ester having a branched-chain alkyl group having 8 or more and 12 or less carbon atoms or a salt thereof, more preferably a sulfosuccinic acid branched alkyl ester having a branched-chain alkyl group having 9 or 10 carbon atoms or a salt thereof, and further preferably a sulfosuccinic acid branched alkyl ester having a branched-chain alkyl group having 10 carbon atoms or a salt thereof. The sulfosuccinic acid ester or a salt thereof is a sulfosuccinic acid branched dialkyl ester or a salt thereof and is preferably a sulfosuccinic acid branched dialkyl ester in which two branched-chain alkyl groups are each a branched-chain alkyl group having 8 or more and 12 or less carbon atoms or a salt thereof, more preferably sulfosuccinic acid branched dialkyl ester in which two branched-chain alkyl groups are each a branched-chain alkyl group having 9 or 10 carbon atoms or a salt thereof, further preferably a sulfosuccinic acid branched dialkyl ester in which two branched-chain alkyl groups are each a branched-chain alkyl group having 10 carbon atoms or a salt thereof, and further preferably bis-(2-propylheptyl) sulfosuccinate or a salt thereof.

Examples of the salt include an alkali metal salt and an alkanolamine salt, and preferred is an alkali metal salt or an alkanolamine salt, more preferred is a salt selected from the group consisting of a sodium salt, a potassium salt, a triethanolamine salt, a diethanolamine salt, and a monoethanolamine salt, and further preferred is a sodium salt.

Examples of the sulfosuccinic acid ester or a salt thereof include compounds of the following Formula 1:

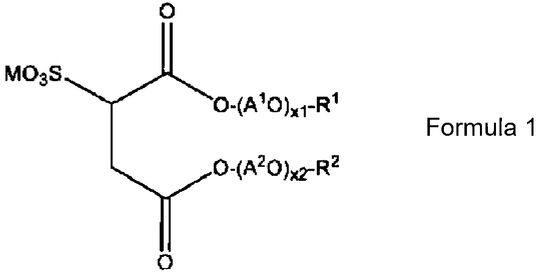

[in Formula 1, R¹ and R² are each a branched-chain alkyl group having 8 or more and 12 or less carbon atoms; A¹O and A²O are each an alkyleneoxy group having 2 or more and 4 or less carbon atoms; x1 and x2 are average addition mole numbers and are each a number of 0 or more and 10 or less; and M is a cation].

In Formula 1, R¹ and R² are each preferably a branched-chain alkyl group selected from the group consisting of a branched-chain nonyl group, a branched-chain decyl group, and a branched-chain dodecyl group and more preferably a branched-chain decyl group. The branched-chain decyl group is preferably a 2-propylheptyl group.

In Formula 1, A¹O and A²O are each an alkyleneoxy group having 2 or more and 4 or less carbon atoms and, preferably 2 or 3 carbon atoms from the viewpoint of lubricity to water. In Formula 1, x1 and x2 represent the average addition mole numbers of A¹O and A²O, respectively, and are each a number of 0 or more and 10 or less and, preferably 6 or less, more preferably 4 or less, and further preferably 2 or less, and are further preferably 0 from the viewpoint of lubricity to water.

In Formula 1, M is a cation. M is preferably a cation other than a hydrogen ion. Examples of M include an alkali metal ion such as a lithium ion, a sodium ion, and a potassium ion; an alkaline earth metal ion such as a calcium ion and a barium ion; and an organic ammonium ion such as a triethanolammonium ion, a diethanolammonium ion, a monoethanolammonium ion, a trimethylammonium ion, and a monomethylammonium ion.

M is preferably an alkali metal ion or an alkanolammonium ion, more preferably a sodium ion, a potassium ion, a triethanolammonium ion, a diethanolammonium ion, or a monoethanolammonium ion, and further preferably a sodium ion from the viewpoint of dispersibility in water.

The sulfosuccinic acid ester or a salt thereof is preferably a compound of the following Formula 1-1. The compound of Formula 1-1 is a compound with x1 and x2 in Formula 1 being each 0.

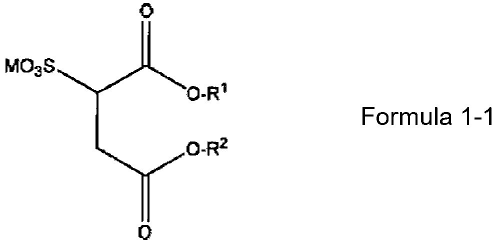

[in Formula 1-1, R¹ and R² are each a branched-chain alkyl group having 8 or more and 12 or less carbon atoms; and M is a cation].

Specific examples and preferable examples of R¹, R², and M in Formula 1-1 are the same as those in Formula 1.

In one preferable embodiment, the sulfosuccinic acid ester or a salt thereof is bis-(2-propylheptyl) sulfosuccinate or a salt thereof.

The amount of the sulfosuccinic acid ester or a salt thereof blended in the cleansing composition is preferably 0.01 mass% or more and more preferably 0.1 mass% or more and is preferably 30.0 mass% or less, more preferably 10.0 mass% or less, and further preferably 2.0 mass% or less. The amount is preferably from 0.01 to 30.0 mass%, more preferably from 0.1 to 10.0 mass%, and further preferably from 0.1 to 2.0 mass%.

The cleansing composition can include various enzymes in addition to the lipase variant of the present invention, such as a hydrolase, an oxidase, a reductase, a transferase, a lyase, an isomerase, a ligase, and a synthetase. Among these enzymes, preferred are a lipase different from the lipase variant of the present invention, an amylase, a protease, a cellulase, a keratinase, an esterase, a cutinase, a pullulanase, a pectinase, a mannanase, a glucosidase, a glucanase, a cholesterol oxidase, a peroxidase, and a laccase, and particularly preferred are a protease, a cellulase, an amylase, and a lipase different from the lipase variant of the present invention.

Examples of the protease include commercially available Alcalase, Esperase, Everlase, Savinase, Kannase, Progress Uno (registered trademark, Novozymes A/S), PREFERENZ, EFFECTENZ, EXCELLENZ (registered trademark, DuPont de Nemours, Inc.), Lavergy (registered trademark, BASF SE), and KAP (Kao Corporation). Alternative examples thereof include each variant of KP43 protease described in JP-A-2020-145938 and Japanese Patent Application No. 2022-118096.

Examples of the cellulase include Celluclean, Carezyme (registered trademark, Novozymes A/S), KAC, and an alkaline cellulase produced by *Bacillus* sp. KSM-S237 strain described in JP-A-10-313859 and a variant alkaline cellulase described in JP-A-2003-313592 (Kao Corporation).

Examples of the amylase include Termamyl, Duramyl, Stainzyme, Stainzyme Plus, Amplify Prime (registered trademark, Novozymes A/S), PREFERENZ, EFFECTENZ (registered trademark, DuPont de Nemours, Inc.), and KAM (Kao Corporation). Alternative examples thereof include each variant of YR288 amylase described in JP-A-2022-60158 and WO2023/176970. Alternative examples thereof include each variant of amylase described in JP-A-2019-500058 and JP-A-2018-516553.

Examples of the lipase include Lipolase and Lipex (registered trademark, Novozymes A/S).

The cleansing composition can include one or a combination of two or more of the enzymes described above, in addition to the lipase variant of the present invention. Examples of the form of combined use include, but not limited to, combined use of the lipase variant of the present invention and the protease; combined use of the lipase variant of the present invention and the amylase; combined use of the lipase variant of the present invention, the protease, and the amylase; combined use of the lipase variant of the present invention, the lipase different from the lipase variant of the present invention, and the protease; combined use of the lipase variant of the present invention, the lipase different from the lipase variant of the present invention, and the amylase; and combined use of the lipase variant of the present invention, the lipase different from the lipase variant of the present invention, the protease, and the amylase, among which combined use of the lipase variant of the present invention, the protease, and the amylase is preferred.

The cleansing composition can include a known detergent component, and examples of the known detergent component include the following.

### (1) Surfactant

The surfactant is blended in an amount of from 0.5 to 90 mass% in the cleansing composition, in particular, preferably in an amount of from 10 to 45 mass% in a powder cleansing composition and in an amount of from 20 to 90 mass% in a liquid cleansing composition. When the cleansing composition is a laundry clothes detergent or an automatic dishwasher detergent, the surfactant is blended generally in an amount of from 0.5 to 20 mass%, preferably from 1 to 15 mass%, more preferably from 1.5 to 10 mass%, further more preferably from 2 to 8 mass%.

Examples of the surfactant that is used in the cleansing composition include, for example, one or a combination of an anionic surfactant, a nonionic surfactant other than the sulfosuccinic acid ester or a salt thereof, an amphoteric surfactant, and a cationic surfactant, and preferably an amphoteric surfactant.

Examples of the amphoteric surfactant include preferably an amine oxide surfactant or a betaine surfactant, and more preferably a tertiary amine oxide surfactant, sulfobetaine surfactant, or a carbobetaine surfactant. Examples of the tertiary amine oxide surfactant include a tertiary amine oxide surfactant in which one of the groups bound to the nitrogen atom is an alkyl group having 8 or more and 18 or less carbon atoms, preferably an alkyl group having 8 or more and 16 or less carbon atoms, further preferably an alkyl group having 8 or more and 14 or less carbon atoms, which may be interrupted by an amide group or an ester group, and the remaining group is an alkyl group having 1 or more and 3 or less carbon atoms and preferably a methyl group. Suitable examples of sulfobetaine surfactant include a compound having one alkyl group having 10 or more and 18 or less, preferably 16 or less, and more preferably 14 or less carbon atoms; two alkyl groups having 1 or more and 3 or less carbon atoms, preferably methyl groups; and a 3-sulfopropyl group or a 2-hydroxy-3-sulfopropyl group. Preferable examples of the carbobetaine surfactant include preferably a carbobetaine surfactant having one alkyl group having 10 or more and 18 or less, preferably 16 or less, and more preferably 14 or less carbon atoms, which may be interrupted by an amide group or an ester group; two alkyl groups having 1 or more and 3 or less carbon atoms, preferably methyl groups; and one carboxyalkyl group, preferably a carboxymethyl group.

### (2) Divalent metal ion scavenger

A divalent metal ion scavenger is blended in an amount of from 0.01 to 50 mass%, preferably from 5 to 40 mass%. Examples of the divalent metal ion scavenger that is used in the cleansing composition include a condensed phosphate such as a tripolyphosphate, a pyrophosphate, and an orthophosphate; an aluminosilicate such as zeolite; and a synthetic layered crystalline silicate, a nitrilotriacetate, an ethylenediaminetetraacetate, a citrate, an isocitrate, and a polyacetal carboxylate. Among these scavengers, a crystalline aluminosilicate (synthetic zeolite) is particularly preferable, and among A-type, X-type, and P-type zeolite, A-type is particularly preferable. Synthetic zeolite having an average primary particle diameter of from 0.1 to 10 µm, particularly, from 0.1 to 5 µm is suitably used.

### (3) Alkali agent

An alkali agent is blended in an amount of from 0.01 to 80 mass%, preferably from 1 to 40 mass%. Examples of the alkali agent that is used in a powder detergent include alkali metal carbonates such as sodium carbonate, collectively known as dense ash or light ash; and amorphous alkali metal silicates such as JIS Nos. 1, 2, and 3. These inorganic alkali agents are effective for particle skeleton formation during drying of the detergent and can provide a detergent that is relatively hard and has excellent fluidity. Other examples of the alkali agent include sodium sesquicarbonate and sodium hydrogen carbonate, and phosphates, such as tripolyphosphate, also have a function as an alkali agent. Examples of the alkali agent that is used in a liquid detergent include, in addition to the above-mentioned alkali agents, sodium hydroxide and mono-, di-, or tri-ethanolamine, and the alkali agent can also be used as a counterion of a surfactant.

### (4) Anti-resoiling agent

An anti-resoiling agent is blended in an amount of from 0.001 to 10 mass%, preferably from 1 to 5 mass%. Examples of the anti-resoiling agent that is used in the cleansing composition include polyethylene glycol, a carboxylic acid polymer, polyvinyl alcohol, and polyvinylpyrrolidone. Among these agents, the carboxylic acid polymer has, in addition to resoiling-preventing ability, a function of capturing metal ions and a function of dispersing solid particle soil from clothing into the wash bath. The carboxylic acid polymer is a homopolymer or a copolymer of acrylic acid, methacrylic acid, itaconic acid, or the like. A copolymer of the above-mentioned monomer and maleic acid is suitable, and the molecular weight is preferably from several thousands to 100 000. In addition to the above-mentioned carboxylic acid polymer, polymers such as polyglycidylate, cellulose derivatives such as carboxymethyl cellulose, and aminocarboxylic acid polymer such as polyaspartic acid are also preferable because of their metal ion scavenging, dispersing, and resoiling-preventing ability.

### (5) Bleaching agent

For example, a bleaching agent such as hydrogen peroxide and percarbonate is preferably blended in an amount of from 1 to 10 mass%. When a bleaching agent is used, tetraacetylethylenediamine (TAED) and a bleaching activator (activator) described in, for example, JP-A-6-316700 can be blended in an amount of from 0.01 to 10 mass%.

### (6) Fluorescent agent

Examples of a fluorescent agent that is used in the cleansing composition include a biphenyl fluorescent agent (e.g., Tinopal CBS-X) and a stilbene fluorescent agent (e.g., DM-type fluorescent dye). The fluorescent agent is preferably blended in an amount of from 0.001 to 2 mass%.

### (7) Chelating agent

The chelating agent is blended for, for example, easy washing of stains or reduction in water hardness upon washing.

Examples of the chelating agent include aminocarboxylic acid-based chelating agents, phosphonic acid-based chelating agents, hydroxycarboxylic acid-based chelating agents, and polyvalent carboxylic acid-based chelating agents.

Examples of the aminocarboxylic acid-based chelating agent include ethylenediaminetetraacetic acid (EDTA), nitrilotriacetic acid (NTA), hydroxyethylethylenediaminetriacetic acid (HEDTA), diethylenetriaminepentaacetic acid (DTPA), methylglycinediacetic acid (MGDA), triethylenetetraminehexaacetic acid (TTHA), glutamic acid diacetate (GLDA), hydroxyethyliminodiacetic acid (HIDA), dihydroxyethylglycine (DHEG), aspartic acid diacetate (ASDA), ethylenediaminesuccinic acid (EDDS), and their salts.

Examples of the phosphonic acid-based chelating agent include hydroxyethylidenediphosphonic acid (HEDP), nitrilotrismethylenephosphonic acid (NTMP), phosphonobutanetricarboxylic acid (PBTC), ethylenediaminetetramethylenephosphonic acid (EDTMP), and their salts.

Examples of the hydroxycarboxylic acid-based chelating agent include citric acid, malic acid, tartaric acid, gluconic acid, lactic acid, and their salts.

Examples of the polyvalent carboxylic acid-based chelating agent include succinic acid, oxalic acid, glutaric acid, adipic acid, fumaric acid, malonic acid, and their salts.

### (8) Other component

The cleansing composition can contain a solvent, a builder, a softener, a reducing agent (such as a sulfite), a foam inhibitor (such as silicone), a fragrance, an antibacterial and antifungal agent (Proxel [tradename], such as benzoic acid), and other additives that are known in the detergent field.

Examples of the solvent include a monovalent alcohol having 1 or more and 3 or less carbon atoms; a multivalent alcohol having 2 or more and 4 or less carbon atoms; a di- or tri-alkylene glycol of which the alkylene glycol unit has from 2 to 4 carbon atoms; and a monoalkoxy (methoxy, ethoxy, propoxy, or butoxy), phenoxy, or benzoxy ether of di- to tetra-alkylene glycols in which the alkylene glycol unit has from 2 to 4 carbon atoms.

The solvent is preferably a water-soluble organic solvent having 2 or more, preferably 3 or more, carbon atoms and having 10 or less, preferably 8 or less, carbon atoms. Here, the water-soluble organic solvent refers to a solvent having octanol/water partition coefficient (LogPow) of 3.5 or less.

Specifically, examples of the solvent include ethanol, isopropyl alcohol; ethylene glycol, propylene glycol, glycerin, isoprene glycol; diethylene glycol, dipropylene glycol; propylene glycol monomethyl ether, propylene glycol monoethyl ether, diethylene glycol monobutyl ether (also referred to as butyl diglycol), phenoxy ethanol, phenoxy triethylene glycol, and phenoxy isopropanol. The solvent is preferably selected from the group consisting of ethanol, propylene glycol, dipropylene glycol, diethylene glycol monobutyl ether, phenoxy ethanol, phenyl glycol, and phenoxy isopropanol. The solvent preferably includes an alkoxy group and further preferably includes at least one or more selected from monoalkoxy, phenoxy, or benzoxy ether of di- to tetra-alkylene glycol in which the alkylene glycol unit has from 2 to 4 carbon atoms. The solvent more preferably contains diethylene glycol monobutyl ether.

The cleansing composition preferably has a water content of from 60 to 99 mass% and/or a larger content of a solvent than that of a surfactant, more preferably a water content of from 60 to 99 mass% and a larger content of a solvent than that of a surfactant.

The cleansing composition can be produced by combining the lipase variant of the present invention obtained by the above method and the above known washing components according to a common method. The form of the detergent can be selected depending on the purpose and can be, for example, a liquid, a powder, a granule, a paste, or a solid.

The thus-obtained cleansing composition can be used as, for example, a clothes detergent, a dishwashing detergent, a bleaching agent, a detergent for washing hard surfaces, a drain pipe detergent, an artificial tooth detergent, or a bactericidal detergent for medical instruments, preferably a clothes detergent or a dishwashing detergent, and more preferably a laundry clothes detergent (laundry detergent), a dishwashing detergent for hand washing, or an automatic dishwasher detergent.

The cleansing composition is suitable for use at 40°C or less, 35°C or less, 30°C or less, or 25°C or less and at 5°C or more, 10°C or more, or 15°C or more and is suitable for use at from 5°C to 40°C, from 10°C to 35°C, from 15°C to 30°C, or from 15°C to 25°C. Examples of the preferable mode of use include use for low-temperature (from 15°C to 30°C) washing at laundries, use for low-temperature (from 15°C to 30°C) dishwashing by hand, and use for low-temperature (from 15°C to 30°C) washing by automatic dishwashers.

The use of the cleansing composition of the present invention enables washing an object to be washed (for example, clothing, tableware, hard surfaces, drain pipes, artificial teeth, and medical instruments) that require the removal of stains, i.e., enables removing stains. Such a washing method includes bringing an object to be washed that requires the removal of stains into contact with the cleansing composition of the present invention. Preferably, the stains are stains containing oil. Examples thereof include oil stains and combined stains containing proteins and carbohydrates in addition to oils.

In the washing method of the present invention, in order to bring an object to be washed and a cleansing composition into contact with each other, the object may be soaked in water in which the cleansing composition has been dissolved, or the cleansing composition may be directly applied to the object. In the method of the present invention, the object to be washed after soaking or application of a cleansing composition may be further hand washed, scrubbed with sponge, or washed with a washing machine, which is not necessarily needed.

Regarding the above-described embodiments, in the present invention, the following aspects are further disclosed.
<1> A lipase variant, comprising one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase variant is a variant of a parent lipase and the parent lipase or the lipase variant has a sequence identity of at least 80% with the amino acid sequence of SEQ ID NO: 4 or 6.
<2> The lipase variant according to <1>, wherein each of the substitutions of the amino acid residues at positions selected from the group consisting of the positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 is a corresponding substitution selected from L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T274I/Q, K278A, and R286M.
<3> The lipase variant according to <2>, comprising any one of the following substitutions (A) to (D):
   (A) N272C/G/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, T2741/Q, K278A, and R286M numbered according to SEQ ID NO: 4,
   (B) V220H substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4,
   (C) V260A/C/F/G/L/M/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, L266P, V269I/L/M, N272C/G/T, T274I/Q, K278A, and R286M numbered according to SEQ ID NO: 4, and
   (D) L208C substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4.
<4> The lipase variant according to <2>, comprising any one of the following substitutions (A') to (D'):
   (A') N272C/G substitution and one or more substitutions selected from the group consisting of L16F, G22M, Y29F, Y43W, L50I/V, S120A/Q, K123I/T, R126E, L142F, G156A, A181K, Q186E, K188Q, L190F, D191V, L208C, M212L, V220H, A221H, L223V, A224Q, F225T, L228W, R233K/T, F234W, S256N, V260A/C/L, V269I/L, T274Q, and K278A numbered according to SEQ ID NO: 4,
   (B') V220H substitution and one or more substitutions selected from the group consisting of L16F, G22C/M, Y43W, L50I/V, S120Q, K123I/T, R126E, L142F, G156A, A181K, Q186E, K188Q, L190F, D191A/E/V, L208C, M212L, A221H, L223V, A224Q, F225T, L228W, R233K/Q, F234W, S256N, V260A/C/L, V269I/L/M, N272C/G, T274Q, and K278A numbered according to SEQ ID NO: 4,
   (C') V260A/C/L substitution and one or more substitutions selected from the group consisting of L16F, G22M, T46Q, L50I/V, K123I, R126E, L142F, G156A, L190F, D191A/E, L208C, M212L, V220H, A224F/Q/Y, F225T, L228W, R233K/Q, R234W, V269I/M, N272C/G, and K278A numbered according to SEQ ID NO: 4, and
   (D') L208C substitution and one or more substitutions selected from the group consisting of L16F, G22C, L50I/V, K123I, R126E, K188Q, L190F, D191A, M212L, V220H, L223V, A224Q/Y, F225T, L228W, R233K, F234W, S256N, V260A/C/L, V269I, N272C/G, K278A, and R286M numbered according to SEQ ID NO: 4.
<5> The lipase variant according to <4>, wherein the (A') to (D') described above are the following:
   (A') N272C substitution and one or more substitutions selected from the group consisting of L16F, G22M, Y29F, Y43W, L50I/V, S120A/Q, K123I, R126E, L142F, G156A, A181K, K188Q, D191V, L208C, V220H, A221H, L223V, A224Q, F225T, R233K/T, F234W, S256N, V260A/C/L, V269I, and T274Q numbered according to SEQ ID NO: 4,
   (B') V220H substitution and one or more substitutions selected from the group consisting of G22C/M, L50I/V, K123I/T, R126E, L142F, G156A, A181K, Q186E, D191V, L208C, M212L, A221H, L223V, L228W, R233K/Q, F234W, S256N, V260C/L, V269I/L/M, N272C/G, T274Q, and K278A numbered according to SEQ ID NO: 4,
   (C') V260C substitution and one or more substitutions selected from the group consisting of L50I/V and L142F numbered according to SEQ ID NO: 4, or (C') V260L substitution and one or more substitutions selected from the group consisting of G22M, L50I/V, G156A, D191A/E, L208C, M212L, V220H, A224F/Q/Y, L228W, V269I/M, and N272G numbered according to SEQ ID NO: 4, and
   (D') L208C substitution and one or more substitutions selected from the group consisting of L16F, G22C, R126E, K188Q, D191A, V220H, L223V, A224Q/Y, L228W, S256N, V260A/C/L, V269I, N272C/G, K278A, and R286M numbered according to SEQ ID NO: 4.
<6> The lipase variant according to <4>, wherein the (A') to (D') described above are the following:
   (A') N272C substitution and one or more substitutions selected from the group consisting of G22M, K123I, G156A, K188Q, L208C, V220H, A221H, L223V, A224Q, F225T, R233T, S256N, V260A/C/L, and V269I numbered according to SEQ ID NO: 4,
   (B') V220H substitution and one or more substitutions selected from the group consisting of G22C/M, G156A, L208C, M212L, L228W, S256N, V260C/L, V269I/L/M, and N272C/G numbered according to SEQ ID NO: 4,
   (C') V260C substitution and one or more substitutions selected from the group consisting of L50I/V and L142F numbered according to SEQ ID NO: 4, or V260L substitution and one or more substitutions selected from the group consisting of G22M, G156A, L208C, V220H, L228W, V269I/M, and N272G numbered according to SEQ ID NO: 4, and **(D')** L208C substitution and one or more substitutions selected from the group consisting of L16F, G22C, K188Q, V220H, L228W, S256N, V260A/C/L, V269I, and N272C/G numbered according to SEQ ID NO: **4.**
<7> The lipase variant according to <2>, comprising at least V220H substitution and N272C/G substitution or V260L substitution and V269I substitution numbered according to SEQ ID NO: **4.**
<8> The lipase variant according to <2>, comprising at least L208C substitution, V220H substitution, and N272C substitution; L142F substitution, V220H substitution, and N272G substitution; or V260L substitution, V269I substitution, and N272G substitution numbered according to SEQ ID NO: 4.
<9> The lipase variant according to <2>, comprising at least L208C substitution, V220H substitution, V269I substitution, and N272C substitution; L50V substitution, L142F substitution, V220H substitution, and N272G substitution; or L50V substitution, V260L substitution, V269I substitution, and N272G substitution numbered according to SEQ ID **NO:** 4.
<10> The lipase variant according to <2>, comprising at least L208C substitution, V220H substitution, S256N substitution, V269I substitution, and N272C substitution; or L50V substitution, L142F substitution, V220H substitution, V260L substitution, and N272G substitution numbered according to SEQ ID NO: 4.
<11> The lipase variant according to <1> or <2>, comprising any one of the substitution or a combination of the substitutions shown in Tables 1-1 to 1-5 and Tables 2-1 to 2-3 described above.
<12> The lipase variant according to any one of <1> to <11>, wherein when the parent lipase or the lipase variant has a sequence identity of at least 80% with the amino acid sequence of SEQ ID NO: 6, each of the substitutions of the amino acid residues at positions selected from the group consisting of the positions corresponding to positions L16, G22, Y29, Y43, A45, T46, L50, S120, K123, R126, T134, L142, A145, G156, K174, A181, K188, L190, D191, L208, H209, M212, L215, V220, A221, L223, A224, F225, L228, R233, F234, S256, V260, L266, V269, N272, T274, K278, and R286 numbered according to SEQ ID NO: 4 is a corresponding substitution selected from L17F, G23C/M, Y30F, Y44W, A46V, T476Q, L51I/M/V, S121A/Q, T124I, R127E, T135A, L143F, A146C/Q, G157A, K175D, A182K, K189Q, L191F, V192A/E, L209C, H210K, A213L, L216C, V221H, A222H, L224A/E/S/V, A225F/Q/Y, F226T, L229W, R234K/Q/T, F235W, S257N, V261A/C/F/G/L/M/T, L267P, V270I/L/M, N273C/G/T, T275I/Q, Q279A, and R287M, numbered according to SEQ ID NO: 6.
<13> The lipase variant according to any one of <1> to <12>, wherein the lipase has improved stability compared to the parent lipase.
<14> A polynucleotide encoding the lipase variant according to any one of <1> to <13>.
<15> A vector or DNA fragment comprising the polynucleotide according to <14>.
<16> A transformed cell comprising the vector or DNA fragment according to <15>.
<17> The transformed cell according to <16>, wherein the transformed cell is a microorganism.
<18> The transformed cell according to <17>, wherein the transformed cell is *Escherichia coli* or a *Bacillus* bacterium, preferably a *Bacillus* bacterium, and more preferably *Bacillus subtilis.*
<19> A method for producing a lipase variant, comprising a step of culturing the transformed cell according to any one of <16> to <18>.
<20> A cleansing composition comprising the lipase variant according to any one of <1> to <13>.
<21> The cleansing composition according to <20>, further comprising a sulfosuccinic acid ester or a salt thereof, preferably a sulfosuccinic acid branched alkyl ester having a branched-chain alkyl group having 8 or more and 12 or less carbon atoms or a salt thereof, more preferably a sulfosuccinic acid branched alkyl ester having a branched-chain alkyl group having 9 or 10 carbon atoms or a salt thereof, and further preferably a sulfosuccinic acid branched alkyl ester having a branched-chain alkyl group having 10 carbon atoms or a salt thereof.
<22> The cleansing composition according to <20>, further comprising a sulfosuccinic acid diester or a salt thereof, preferably a sulfosuccinic acid branched dialkyl ester in which two branched-chain alkyl groups are each a branched-chain alkyl group having 8 or more and 12 or less carbon atoms or a salt thereof, more preferably a sulfosuccinic acid branched dialkyl ester in which two branched-chain alkyl groups are each a branched-chain alkyl group having 9 or 10 carbon atoms or a salt thereof, further preferably a sulfosuccinic acid branched dialkyl ester in which two branched-chain alkyl groups are each a branched-chain alkyl group having 10 carbon atoms or a salt thereof, and further preferably bis-(2-propylheptyl) sulfosuccinate or a salt thereof.
<23> The cleansing composition according to any one of <20> to <22>, wherein a content of water is from 60 to 90 mass%, and/or a content of a solvent is larger than that of a surfactant, and preferably, a content of water is from 60 to 90 mass%, and a content of a solvent is larger than that of a surfactant.
<24> The cleansing composition according to any one of <20> to <23> which is a clothes detergent or a dishwashing detergent.
<25> The cleansing composition according to any one of <20> to <24> which is a powder or a liquid.
<26> A stain-washing method comprising using the cleansing composition according to any one of <20> to <25>.
<27> The method according to <26>, comprising bringing an object to be washed into contact with the cleansing composition according to any one of <20> to <25>.
<28> Use of the lipase variant according to any one of <1> to <13> for producing a cleansing composition.
<29> The use of the lipase variant according to any one of <1> to <13> for washing a stain.
<30> A method for improving stability of a lipase, comprising the step of substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase or a lipase after amino acid residue substitution of the lipase has a sequence identity of at least 80% with the amino acid sequence of SEQ ID NO: 4 or 6.
<31> A method for producing a variant of a parent lipase, comprising the step of substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase or the lipase variant has a sequence identity of at least 80% with the amino acid sequence of SEQ ID NO: 4 or 6.
<32> The method according to <30> or <31>, wherein each of the substitutions of the amino acid residues at positions selected from the group consisting of the positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 is a corresponding substitution selected from L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M.
<33> The method according to <32>, wherein the substitution comprises any one of the following substitutions (A) to (D):
   (A) N272C/G/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, T274I/Q, K278A, and R286M numbered according to SEQ ID NO: 4,
   (B) V220H substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4,
   (C) V260A/C/F/G/L/M/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, L266P, V269I/L/M, N272C/G/T, T2741/Q, K278A, and R286M numbered according to SEQ ID NO: 4, and
   (D) L208C substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4.
<34> The method according to <32>, wherein the substitution comprises any one of the following substitutions (A') to (D'):
   (A') N272C/G substitution and one or more substitutions selected from the group consisting of L16F, G22M, Y29F, Y43W, L50I/V, S120A/Q, K123I/T, R126E, L142F, G156A, A181K, Q186E, K188Q, L190F, D191V, L208C, M212L, V220H, A221H, L223V, A224Q, F225T, L228W, R233K/T, F234W, S256N, V260A/C/L, V269I/L, T274Q, and K278A numbered according to SEQ ID NO: 4,
   (B') V220H substitution and one or more substitutions selected from the group consisting of L16F, G22C/M, Y43W, L50I/V, S120Q, K123I/T, R126E, L142F, G156A, A181K, Q186E, K188Q, L190F, D191A/E/V, L208C, M212L, A221H, L223V, A224Q, F225T, L228W, R233K/Q, F234W, S256N, V260A/C/L, V269I/L/M, N272C/G, T274Q, and K278A numbered according to SEQ ID NO: 4,
   (C') V260A/C/L substitution and one or more substitutions selected from the group consisting of L16F, G22M, T46Q, L50I/V, K123I, R126E, L142F, G156A, L190F, D191A/E, L208C, M212L, V220H, A224F/Q/Y, F225T, L228W, R233K/Q, R234W, V269I/M, N272C/G, and K278A numbered according to SEQ ID NO: 4, and
   (D') L208C substitution and one or more substitutions selected from the group consisting of L16F, G22C, L50I/V, K123I, R126E, K188Q, L190F, D191A, M212L, V220H, L223V, A224Q/Y, F225T, L228W, R233K, F234W, S256N, V260A/C/L, V269I, N272C/G, K278A, and R286M numbered according to SEQ ID NO: 4.
<35> The method according to <34>, wherein the (A') to (D') described above are the following:
   (A') N272C substitution and one or more substitutions selected from the group consisting of L16F, G22M, Y29F, Y43W, L50I/V, S120A/Q, K123I, R126E, L142F, G156A, A181K, K188Q, D191V, L208C, V220H, A221H, L223V, A224Q, F225T, R233K/T, F234W, S256N, V260A/C/L, V269I, and T274Q numbered according to SEQ ID NO: 4,
   (B') V220H substitution and one or more substitutions selected from the group consisting of G22C/M, L50I/V, K123I/T, R126E, L142F, G156A, A181K, Q186E, D191V, L208C, M212L, A221H, L223V, L228W, R233K/Q, F234W, S256N, V260C/L, V269I/L/M, N272C/G, T274Q, and K278A numbered according to SEQ ID NO: 4,
   (C') V260C substitution and one or more substitutions selected from the group consisting of L50I/V and L142F numbered according to SEQ ID NO: 4, or (C') V260L substitution and one or more substitutions selected from the group consisting of G22M, L50I/V, G156A, D191A/E, L208C, M212L, V220H, A224F/Q/Y, L228W, V269I/M, and N272G numbered according to SEQ ID NO: 4, and
   (D') L208C substitution and one or more substitutions selected from the group consisting of L16F, G22C, R126E, K188Q, D191A, V220H, L223V, A224Q/Y, L228W, S256N, V260A/C/L, V269I, N272C/G, K278A, and R286M numbered according to SEQ ID NO: 4.
<36> The method according to <34>, wherein the (A') to (D') described above are the following:
   (A') N272C substitution and one or more substitutions selected from the group consisting of G22M, K123I, G156A, K188Q, L208C, V220H, A221H, L223V, A224Q, F225T, R233T, S256N, V260A/C/L, and V269I numbered according to SEQ ID NO: 4,
   (B') V220H substitution and one or more substitutions selected from the group consisting of G22C/M, G156A, L208C, M212L, L228W, S256N, V260C/L, V269I/L/M, and N272C/G numbered according to SEQ ID NO: 4,
   **(C')** V260C substitution and one or more substitutions selected from the group consisting of L50I/V and L142F numbered according to SEQ ID NO: 4, or V260L substitution and one or more substitutions selected from the group consisting of G22M, G156A, L208C, V220H, L228W, V269I/M, and N272G numbered according to SEQ ID NO: 4, and (D') L208C substitution and one or more substitutions selected from the group consisting of L16F, G22C, K188Q, V220H, L228W, S256N, V260A/C/L, V269I, and N272C/G numbered according to SEQ ID NO: 4.
<37> The method according to <32>, wherein the substitution comprises at least V220H substitution and N272C/G substitution or V260L substitution and V269I substitution numbered according to SEQ ID NO: 4.
<38> The method according to <32>, wherein the substitution comprises at least L208C substitution, V220H substitution; and N272C substitution, L142F substitution, V220H substitution, and N272G substitution; or V260L substitution, V269I substitution, and N272G substitution numbered according to SEQ ID **NO:** 4.
<39> The method according to <32>, wherein the substitution comprises at least L208C substitution, V220H substitution, V269I substitution, and N272C substitution; L50V substitution, L142F substitution, V220H substitution, and N272G substitution; or L50V substitution, V260L substitution, V269I substitution, and N272G substitution numbered according to SEQ ID NO: 4.
<40> The method according to <32>, wherein the substitution comprises at least L208C substitution, V220H substitution, S256N substitution, V269I substitution, and N272C substitution; or L50V substitution, L142F substitution, V220H substitution, V260L substitution, and N272G substitution numbered according to SEQ ID NO: 4.
<41> The method according to any one of <30> to <32>, wherein the substitution comprises any one of the substitution or a combination of the substitutions shown in Tables 1-1 to 1-5 and Tables 2-1 to 2-3 described above.
<42> The method according to any one of <30> to <41>, wherein when the lipase or a lipase after amino acid residue substitution of the lipase has a sequence identity of at least 80% with the amino acid sequence of SEQ ID NO: 6, each of the substitutions of the amino acid residues at positions selected from the group consisting of the positions corresponding to positions L16, G22, Y29, Y43, A45, T46, L50, S120, K123, R126, T134, L142, A145, G156, K174, A181, K188, L190, D191, L208, H209, M212, L215, V220, A221, L223, A224, F225, L228, R233, F234, S256, V260, L266, V269, N272, T274, K278, and R286 numbered according to SEQ ID NO: 4 is a corresponding substitution selected from L17F, G23C/M, Y30F, Y44W, A46V, T476Q, L51I/M/V, S121A/Q, T124I, R127E, T135A, L143F, A146C/Q, G157A, K175D, A182K, K189Q, L191F, V192A/E, L209C, H210K, A213L, L216C, V221H, A222H, L224A/E/S/V, A225F/Q/Y, F226T, L229W, R234K/Q/T, F235W, S257N, V261A/C/F/G/L/M/T, L267P, V270I/L/M, N273C/G/T, T275I/Q, Q279A, and R287M, numbered according to SEQ ID NO: 6.

### Examples

The present invention will be described in further detail based on Examples below, but is not limited thereto.

### Example 1

### (1) Construction of lipase expression plasmid

Plasmids pHY-CnLipA and pHY-CspLipA were constructed by using a plasmid pHY-S237 described in Example 7 of WO 2006/068148A1 as a template and transferring an artificially synthesized gene of CnLip I44T L120S S130A A134T A135V L136M S137N (hereinafter, referred to as CnLipA; the polynucleotide of SEQ ID NO: 1 which encodes the amino acid sequence of SEQ ID NO: 2) or CspLip I45T L121S S131A A135T A136V S138N A213M (hereinafter, referred to as CspLipA; the polynucleotide of SEQ ID NO: 5 which encodes the amino acid sequence of SEQ ID NO: 6) described in Japanese Patent Application No. 2023-025141 to a full-length ORF of an alkaline cellulase gene by InFusion reaction. A plasmid pHY-CnLipAm for expression of CnLipAm (the polynucleotide of SEQ ID NO: 3 which encodes the amino acid sequence of SEQ ID NO: 4) was prepared by using pHY-CnLipA as a template and introducing thereto five mutations by site-directed mutagenesis by PCR using a complementary primer pair (Zheng, Lei, Ulrich Baumann, and Jean-Louis Reymond. Nucleic Acids Research 32.14 (2004): e115-e115.).

In further mutagenesis to each lipase, the site-directed mutagenesis by PCR using a complementary primer pair was used.

### (2) Preparation of lipase solution

A lipase expression plasmid was introduced into a *Bacillus subtilis* strain 168ΔsigF described in JP-A-2003-47490 by a protoplast method. The strain was cultured in 2 × L-maltose culture medium (2% tryptone, 1% yeast extract, 1% NaCl, 7.5% maltose, 7.5 ppm manganese sulfate pentahydrate, 0.04% calcium chloride dihydrate, and 15 ppm tetracycline: % is (w/v)%) at 30°C for 3 days, and the culture supernatant containing the lipase was then collected by centrifugation.

### (3) Evaluation of stability in model washing solution

4-Nitrophenyl butyrate (SIGMA) was used as a substrate. The activity of a lipase can be determined by measuring the rate of increase in absorbance due to release of 4-nitrophenol by the function of a lipase. A mixture of 4-nitrophenyl butyrate added at a final concentration of 2 mM to 20 mM Tris-HCl (pH 7.0) was used as a substrate solution.

The lipase solution prepared by the method described in the section (2) was added to the model washing solution shown in Table 3, then incubated at 40, 45, 50, 55 or 60°C for each time described in Tables 4 to 20, and then diluted 51-fold with 20 mM Tris-HCl (pH 7.0), and each dilution was used in activity measurement. A deactivation rate per unit time (h) by the treatment at 40, 45, 50, 55, or 60°C was calculated with the activity value of the sample before the treatment at 40, 45, 50, 55, or 60°C as initial activity, and a half-life (h) of each variant was calculated therefrom. Relative stability was determined by dividing the half-life (h) of each variant by the half-life (h) of the parent polypeptide. The results are shown in Tables 4 to 20. Each table shows the relative stability for variants having the mutation of the table added to the parent polypeptide (parent enzyme) described in the top row. All the variants were found to have improved stability compared to the parent polypeptide.

**[Table 3]**

| Blended component | mass% |
|---|---|
| Sulfosuccinic acid branched alkyl ester | 0.2 |
| Betaine | 2 |
| Butyl diglycol | 6.5 |
| Citric acid | 2 |
| NaOH | Added so as to attain pH 6.5 |
| Water | Balance |
| Total | 100 |

| | |
|---|---|
| • Sulfosuccinic acid branched alkyl ester: sodium bis-(2-propylheptyl)sulfosuccinate • Betaine: lauryl (2-hydroxy-3-sulfopropyl)dimethylbetaine, AMPHITOL 20HD, manufactured by Kao Corp. | |

**[Table 4]**

| (Relative stability in treatment at 45°C for 1 hr) | |
|---|---|
| Lipase | Relative stability |
| CnLipAm (parent enzyme) | 1.00 |
| V260A | 1.38 |
| V260C | 2.07 |
| V260G | 3.26 |
| V260L | 1.70 |
| V260M | 2.18 |
| V260F | 5.15 |
| V260T | 1.49 |
| L266P | 2.91 |
| V2691 | 1.39 |
| V269L | 1.06 |
| V269M | 1.89 |
| N272C | 2.94 |
| N272G | 10.47 |
| N272T | 1.92 |
| Y29F | 1.38 |
| L142F | 1.29 |

| Lipase | Relative stability |
|---|---|
| A145C | 4.39 |
| A145Q | 6.63 |
| V220H | 1.50 |
| T274Q | 1.70 |
| T2741 | 1.16 |
| L215C | 1.80 |
| A221H | 2.76 |
| L223A | 1.19 |
| L223E | 1.29 |
| L223S | 1.38 |
| L16F | 1.31 |
| G22C | 2.50 |
| G22M | 1.55 |
| L501 | 1.24 |
| L50M | 1.19 |
| L50V | 1.35 |
| K1231 | 1.12 |
| A224Q | 1.58 |
| K174D | 2.92 |
| S120A | 1.08 |
| K123T | 1.07 |
| A181K | 1.30 |
| S120Q | 1.11 |
| D191V | 1.88 |
| L208C | 1.91 |
| R233T | 1.65 |
| K188Q | 1.59 |
| R126E | 1.54 |
| T134A | 1.26 |

**[Table 5-1]**

| (Relative stability in treatment at 40°C for 3 hr) | |
|---|---|
| Lipase | Relative stability |
| CnLipAm N272C (parent enzyme) | 1.00 |
| Y29F | 1.15 |
| L142F | 1.32 |
| V220H | 1.72 |
| T274Q | 1.31 |
| K1231 | 1.41 |
| A224Q | 1.43 |
| K123T | 1.09 |
| A181K | 1.10 |
| D191V | 1.27 |
| L208C | 2.25 |
| Q186E | 1.08 |
| K188Q | 1.45 |
| R126E | 1.22 |

| Lipase | Relative stability |
|---|---|
| V260A | 2.17 |
| V260C | 4.95 |
| V260L | 1.81 |
| V2691 | 2.34 |
| A221H | 2.78 |
| L16F | 1.30 |
| G22M | 3.75 |
| L501 | 1.23 |
| L50V | 1.22 |
| G156A | 1.52 |
| S120A | 1.15 |
| S120Q | 1.36 |
| R233T | 2.08 |
| S256N | 3.26 |

**[Table 5-2]**

| (Relative stability in treatment at 40°C for 3 hr) | |
|---|---|
| Lipase | Relative stability |
| CnLipAm V220H (parent enzyme) | 1.00 |
| N272C | 3.88 |
| N272G | 6.85 |
| L142F | 1.23 |
| T274Q | 1.20 |
| K1231 | 1.26 |
| K123T | 1.22 |
| A181K | 1.11 |
| D191V | 1.15 |
| L208C | 2.36 |
| Q186E | 1.10 |
| K188Q | 1.09 |
| R126E | 1.28 |

| Lipase | Relative stability |
|---|---|
| V260C | 3.89 |
| V260L | 1.66 |
| V2691 | 2.48 |
| V269L | 3.56 |
| V269M | 4.09 |
| A221H | 1.21 |
| G22C | 2.85 |
| G22M | 2.92 |
| L501 | 1.21 |
| L50V | 1.28 |
| G156A | 1.47 |
| S120Q | 1.08 |
| S256N | 2.37 |

**[Table 5-3]**

| (Relative stability in treatment at 40°C for 3 hr) | |
|---|---|
| Lipase | Relative stability |
| CnLipAm V260C (parent enzyme) | 1.00 |
| L142F | 1.94 |
| A224Q | 1.03 |
| N272G | 1.07 |
| L501 | 1.49 |
| L50V | 1.65 |

**[Table 5-4]**

| (Relative stability in treatment at 40°C for 3 hr) | |
|---|---|
| Lipase | Relative stability |
| CnLipAm V260L (parent enzyme) | 1.00 |
| V220H | 1.90 |
| A224Q | 1.11 |
| L208C | 2.48 |
| N272G | 4.07 |
| V2691 | 2.78 |
| L16F | 1.01 |
| G22M | 6.12 |
| L501 | 1.10 |
| L50V | 1.10 |
| G156A | 2.41 |
| V269M | 4.20 |

**[Table 5-5]**

| (Relative stability in treatment at 40°C for 3 hr) | |
|---|---|
| Lipase | Relative stability |
| CnLipAm L208C (parent enzyme) | 1.00 |
| V220H | 3.26 |
| A224Q | 1.21 |
| K188Q | 1.50 |
| R126E | 1.15 |
| N272G | 3.50 |
| V260A | 1.65 |
| V260C | 1.81 |
| V260L | 1.82 |
| V2691 | 2.13 |
| L16F | 1.54 |
| L501 | 1.02 |
| G22C | 2.15 |
| S256N | 5.27 |

**[Table 6-1]**

| (Relative stability in treatment at 40°C for 5 hr) | |
|---|---|
| Lipase | Relative stability |
| CnLipAm V220H N272C (parent enzyme) | 1.00 |
| L142F | 1.22 |
| K1231 | 1.11 |
| L208C | 2.86 |
| R126E | 1.08 |
| V260A | 1.76 |
| V260L | 1.68 |
| V2691 | 1.41 |
| L16F | 1.04 |
| L50V | 1.39 |
| G156A | 1.19 |
| V269L | 2.77 |

**[Table 6-2]**

| (Relative stability in treatment at 40°C for 5 hr) | |
|---|---|
| Lipase | Relative stability |
| CnLipAm V220H N272G (parent enzyme) | 1.00 |
| L142F | 1.58 |
| K1231 | 1.05 |
| A224Q | 1.69 |
| L208C | 1.09 |
| R126E | 1.01 |
| V260L | 1.32 |
| L16F | 1.23 |
| L501 | 1.19 |
| L50V | 1.10 |

**[Table 6-3]**

| (Relative stability in treatment at 40°C for 5 hr) | |
|---|---|
| Lipase | Relative stability |
| CnLipAm V260L V2691 (parent enzyme) | 1.00 |
| A224Q | 2.52 |
| L208C | 2.38 |
| L501 | 1.82 |
| L50V | 1.85 |
| N272G | 3.73 |

**[Table 7-1]**

| (Relative stability in treatment at 40°C for 16 hr) | |
|---|---|
| Lipase | Relative stability |
| CnLipAm V220H N272C L208C (parent enzyme) | 1.00 |
| K1231 | 1.20 |
| V260L | 1.67 |
| L16F | 1.54 |
| L50V | 1.25 |
| V2691 | 2.65 |

**[Table 7-2]**

| (Relative stability in treatment at 40°C for 16 hr) | |
|---|---|
| Lipase | Relative stability |
| CnLipAm V220H N272G L142F (parent enzyme) | 1.00 |
| K1231 | 1.03 |
| A224Q | 1.13 |
| V260L | 1.50 |
| L16F | 1.07 |
| L50V | 1.29 |

**[Table 8-1]**

| (Relative stability in treatment at 45°C for 5 hr) | |
|---|---|
| Lipase | Relative stability |
| CnLipAm V220H N272G L142F L50V (parent enzyme) | 1.00 |
| K1231 | 1.04 |
| V260L | 1.58 |
| L16F | 1.15 |

**[Table 8-2]**

| (Relative stability in treatment at 45°C for 5 hr) | |
|---|---|
| Lipase | Relative stability |
| CnLipAm V260L V2691 N272G (parent enzyme) | 1.00 |
| A224Q | 1.04 |
| L208C | 1.14 |
| L501 | 1.24 |
| L50V | 1.40 |

**[Table 9]**

| (Relative stability in treatment at 50°C for 16 hr) | |
|---|---|
| Lipase | Relative stability |
| CnLipAm V220H N272C L208C V2691 (parent enzyme) | 1.00 |
| V260L | 1.59 |
| L16F | 2.48 |
| L50V | 1.09 |
| S256N | 2.36 |

**[Table 10-1]**

| (Relative stability in treatment at 40°C for 16 hr) | |
|---|---|
| Lipase | Relative stability |
| CnLipAm V220H N272G L142F L50V V260L (parent enzyme) | 1.00 |
| K123I | 1.16 |
| L16F | 2.39 |
| R126E | 1.41 |

**[Table 10-2]**

| (Relative stability in treatment at 40°C for 16 hr) | |
|---|---|
| Lipase | Relative stability |
| CnLipAm V260L V2691 N272G L50V (parent enzyme) | 1.00 |
| A224Q | 1.39 |
| L208C | 1.17 |
| L501 | 1.03 |
| R126E | 1.09 |

**[Table 11]**

| (Relative stability in treatment at 55°C for 16 hr) | |
|---|---|
| Lipase | Relative stability |
| CnLipAm V220H N272C L208C V2691 S256N (parent enzyme) | 1.00 |
| L50V | 1.18 |
| R126E | 1.54 |

**[Table 12]**

| (Relative stability in treatment at 45°C for 30 min) | |
|---|---|
| Lipase | Relative stability |
| CspLipA (parent enzyme) | 1.00 |
| L209C | 2.50 |
| L51V | 1.69 |
| V261C L17F | 4.74 |
| V221H N273C | 6.24 |
| V221H N273G | 10.25 |
| V261L V2701 | 4.33 |
| V221H N273C L209C | 9.30 |
| V221H N273G L143F L51V | 12.42 |
| V221H N273C L209C S257N R127E | 14.26 |
| V221H N273G L143F L51V V261L T1241 | 32.94 |
| V221H N273C L209C S257N R127E V2701 | 40.69 |

**[Table 13]**

| (Relative stability in treatment at 45°C for 30 min) | |
|---|---|
| Lipase | Relative stability |
| CnLipAm (parent enzyme) | 1.00 |
| K278A | 1.21 |
| M212L | 1.18 |
| R286M | 1.12 |
| Y43W | 1.26 |
| T46Q | 1.38 |
| A45V | 1.33 |
| D191A | 1.39 |
| F234W | 1.36 |
| A224F | 1.29 |
| H209K | 1.22 |
| L223V | 1.84 |
| F225T | 2.11 |

**[Table 14]**

| (Relative stability in treatment at 50°C for 20 min) | |
|---|---|
| Lipase | Relative stability |
| CnLipAm (parent enzyme) | 1.00 |
| L190F | 1.15 |
| D191E | 1.27 |
| R233Q | 1.24 |
| R233K | 1.41 |
| A224Y | 1.02 |
| L228W | 1.18 |

**[Table 15-1]**

| (Relative stability in treatment at 40°C for 3 hr) | |
|---|---|
| Lipase | Relative stability |
| CnLipAm N272C (parent enzyme) | 1.00 |
| M212L | 1.09 |
| Y43W | 1.14 |
| R233K | 1.25 |
| F234W | 1.32 |
| L223V | 1.65 |
| F225T | 1.54 |

**[Table 15-2]**

| (Relative stability in treatment at 40°C for 3 hr) | |
|---|---|
| Lipase | Relative stability |
| CnLipAm V220H (parent enzyme) | 1.00 |
| L190F | 1.02 |
| K278A | 1.13 |
| M212L | 1.52 |
| Y43W | 1.04 |
| D191A | 1.05 |
| D191E | 1.09 |
| R233Q | 1.13 |
| R233K | 1.10 |
| F234W | 1.35 |
| L228W | 1.45 |
| L223V | 1.17 |

**[Table 15-3]**

| (Relative stability in treatment at 40°C for 3 hr) | |
|---|---|
| Lipase | Relative stability |
| CnLipAm V260L (parent enzyme) | 1.00 |
| M212L | 1.11 |
| T46Q | 1.08 |
| D191A | 1.21 |
| D191E | 1.14 |
| R233Q | 1.03 |
| R233K | 1.04 |
| F234W | 1.09 |
| A224F | 1.36 |
| A224Y | 1.12 |
| L228W | 1.41 |

**[Table 15-4]**

| (Relative stability in treatment at 40°C for 3 hr) | |
|---|---|
| Lipase | Relative stability |
| CnLipAm L208C (parent enzyme) | 1.00 |
| K278A | 1.11 |
| R286M | 1.22 |
| D191A | 1.28 |
| A224Y | 1.21 |
| L228W | 1.48 |
| L223V | 1.39 |

**[Table 16-1]**

| (Relative stability in treatment at 40°C for 5 hr) | |
|---|---|
| Lipase | Relative stability |
| CnLipAm V220H N272C (parent enzyme) | 1.00 |
| K278A | 1.12 |
| L190F | 1.53 |
| M212L | 1.55 |
| R233K | 1.08 |
| F234W | 1.09 |
| L228W | 1.48 |

**[Table 16-2]**

| (Relative stability in treatment at 40°C for 5 hr) | |
|---|---|
| Lipase | Relative stability |
| CnLipAm V220H N272G (parent enzyme) | 1.00 |
| R233K | 1.06 |
| F225T | 1.57 |

**[Table 16-3]**

| (Relative stability in treatment at 40°C for 5 hr) | |
|---|---|
| Lipase | Relative stability |
| CnLipAm V260L V2691 (parent enzyme) | 1.00 |
| L190F | 1.29 |
| K278A | 1.43 |
| M212L | 1.74 |
| R233K | 1.60 |

**[Table 17-1]**

| (Relative stability in treatment at 45°C for 16 hr) | |
|---|---|
| Lipase | Relative stability |
| CnLipAm V220H N272G L142F L50V V260L L16F (parent enzyme) | 1.00 |
| K278A | 1.15 |
| M212L | 1.23 |
| R233K | 1.12 |
| F225T | 1.63 |

**[Table 17-2]**

| (Relative stability in treatment at 45°C for 16 hr) | |
|---|---|
| Lipase | Relative stability |
| CnLipAm V260L V2691 N272G L50V A224Q (parent enzyme) | 1.00 |
| K278A | 1.47 |
| M212L | 1.04 |

**[Table 17-3]**

| (Relative stability in treatment at 45°C for 16 hr) | |
|---|---|
| Lipase | Relative stability |
| CnLipAm V220H N272G L142F L50V V260L L16F F225T (parent enzyme) | 1.00 |
| K278A | 1.03 |

**[Table 17-4]**

| (Relative stability in treatment at 45°C for 16 hr) | |
|---|---|
| Lipase | Relative stability |
| CnLipAm V260L V2691 N272G L50V A224Q K278A (parent enzyme) | 1.00 |
| M212L | 1.37 |

**[Table 18]**

| (Relative stability in treatment at 40°C for 7 d) | |
|---|---|
| Lipase | Relative stability |
| CnLipAm V220H N272C L208C V2691 S256N R126E (parent enzyme) | 1.00 |
| K278A | 1.11 |
| F234W | 1.30 |
| F225T | 2.49 |

**[Table 19]**

| (Relative stability in treatment at 60°C for 16 hr) | |
|---|---|
| Lipase | Relative stability |
| CnLipAm V220H N272C L208C V2691 S256N R126E K278A (parent enzyme) | 1.00 |
| M212L | 1.41 |
| L190F | 1.21 |
| R233K | 1.10 |
| L223V | 1.21 |
| F225T | 1.50 |

**[Table 20]**

| (Relative stability in treatment at 40°C for 7 d) | |
|---|---|
| Lipase | Relative stability |
| CnLipAm V220H N272C L208C V2691 S256N R126E K278A M212L (parent enzyme) | 1.00 |
| L190F | 1.14 |
| R233K | 1.36 |
| F234W | 1.24 |
| L228W | 1.30 |
| L223V | 1.45 |
| F225T | 1.46 |

### (4) Evaluation of detergency in model washing solution

The ability to remove triglycerides on a hard surface was evaluated as detergency. Beef tallow (SIGMA, 03-0660) and rapeseed oil (SIGMA, 23-0450) were mixed at a weight ratio of 9 : 1, the mixture was dissolved in triple volumes of chloroform, and the solution was then colored with 0.2 wt% Sudan III to give a model stain. Ten microliter of the model stain was dropwise added to each well bottom of a 96-well polypropylene deep well plate, and the chloroform was evaporated and dried to make a stain plate.

The culture supernatant of the section (2) was diluted with 20 mM Tris-HCl (pH 7.0) to a lipase concentration of 200 mg/L, and the resulting lipase solution was added to the model washing solution described in Table 3 in an amount of 1/50 of the model washing solution. Immersion washing was performed by gently adding 300 µL of the washing solution to each well of the stain plate and leaving it to stand at room temperature (about 22°C) for 15 minutes. One hundred microliter of each washing solution was taken from each well while avoiding contact with the stain on the bottom and was transferred to another 96-well plate. In order to quantitatively measure Sudan III in a model stain that was solubilized in the washing solution by the immersion washing, the absorbance (A500) at 500 nm was measured. A500 corresponds to the release amount of oil into the washing solution and can be used as an indicator of detergency. The enzymatic effect (ΔA500) on detergency was determined by subtracting A500 of a washing solution containing 20 mM Tris-HCl (pH 7.0) instead of the lipase from A500 of the washing solution containing each lipase. The results of washing for 15 minutes are shown in Table 21. The stabilized variant of the present invention was confirmed to have no reduction in detergency.

**[Table 21]**

| Lipase | Δ A500 |
|---|---|
| CnLipAm (parent enzyme) | 0.083 |
| N272C | 0.115 |
| N272G | 0.114 |
| Y29F | 0.096 |
| L142F | 0.082 |
| K1231 | 0.086 |
| A224Q | 0.101 |
| V220H | 0.093 |
| L16F | 0.085 |
| R126E | 0.086 |
| L208C | 0.094 |
| N272C V220H | 0.098 |
| N272C R126E | 0.099 |
| V220H N272C | 0.108 |
| V220H L208C | 0.100 |
| N272C V220H L208C | 0.087 |

### Reference Example 1: Comparison of stability between model washing solution and another formulation

A commercially available liquid detergent (Kao Corp., Attack ZERO (hereinafter, referred to as detergent 1) was left to stand for 30 minutes in a boiling water bath to deactivate the blended enzyme. Savinase (SIGMA, P3111) was added at a final concentration of 0.5% to the detergent and thoroughly mixed. CnLipAm prepared by the method described in the section (2) was added to the model washing solution shown in Table 3 and the detergent 1 containing 0.5% Savinase, then incubated at 45°C for 1 hour, and then diluted 51-fold with 20 mM Tris-HCl (pH 7.0), and each dilution was used in activity measurement. The activity value of the sample before the treatment at 45°C was defined as initial activity, and the ratio of the activity value after the treatment to the initial activity was used as residual activity (%). The results are shown in Table 22. A phenomenon was confirmed in which the lipase was significantly destabilized in the model washing solution described in Table 3 whose suitability for washing was disclosed in Patent Literature 1, even compared to another formulation containing a protease which degrades proteins.

**[Table 22]**

| Formulation | Residual activity (%) |
|---|---|
| Model formulation of Table 3 | 3.61 |
| Detergent 1 + 0.5% Savinase | 67.75 |

## Claims

1. A lipase variant, comprising one or more substitutions of amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase variant is a variant of a parent lipase and the parent lipase or the lipase variant has a sequence identity of at least 80% with the amino acid sequence of SEQ ID NO: 4 or 6.

2. The lipase variant according to claim 1, wherein each of the substitutions of the amino acid residues at positions selected from the group consisting of the positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4 is a corresponding substitution selected from L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M.

3. The lipase variant according to claim 2, comprising any one of the following substitutions (A) to (D):
(A) N272C/G/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, T2741/Q, K278A, and R286M numbered according to SEQ ID NO: 4,
(B) V220H substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4,
(C) V260A/C/F/G/L/M/T substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, L208C, H209K, M212L, L215C, V220H, A221H, L223A/E/S/V, A224F/Q/Y, F225T, L228W, R233K/Q/T, F234W, S256N, L266P, V269I/L/M, N272C/G/T, T2741/Q, K278A, and R286M numbered according to SEQ ID NO: 4, and
(D) L208C substitution and one or more substitutions selected from the group consisting of L16F, G22C/M, Y29F, Y43W, A45V, T46Q, L50I/M/V, S120A/Q, K123I/T, R126E, T134A, L142F, A145C/Q, G156A, K174D, A181K, Q186E, K188Q, L190F, D191A/E/V, H209K, M212L, L215C, V220H, A221H, L223A/E/S/V, A224F/Q/Y, F225T, L228W, R233K/Q/T, F234W, S256N, V260A/C/F/G/L/M/T, L266P, V269I/L/M, N272C/G/T, T2741/Q, K278A, and R286M numbered according to SEQ ID NO: 4.

4. The lipase variant according to claim 2, comprising any one of the following substitutions (A') to (D') :
(A') N272C/G substitution and one or more substitutions selected from the group consisting of L16F, G22M, Y29F, Y43W, L50I/V, S120A/Q, K123I/T, R126E, L142F, G156A, A181K, Q186E, K188Q, L190F, D191V, L208C, M212L, V220H, A221H, L223V, A224Q, F225T, L228W, R233K/T, F234W, S256N, V260A/C/L, V269I/L, T274Q, and K278A numbered according to SEQ ID NO: 4,
(B') V220H substitution and one or more substitutions selected from the group consisting of L16F, G22C/M, Y43W, L50I/V, S120Q, K123I/T, R126E, L142F, G156A, A181K, Q186E, K188Q, L190F, D191A/E/V, L208C, M212L, A221H, L223V, A224Q, F225T, L228W, R233K/Q, F234W, S256N, V260A/C/L, V269I/L/M, N272C/G, T274Q, and K278A numbered according to SEQ ID NO: 4,
(C') V260A/C/L substitution and one or more substitutions selected from the group consisting of L16F, G22M, T46Q, L50I/V, K123I, R126E, L142F, G156A, L190F, D191A/E, L208C, M212L, V220H, A224F/Q/Y, F225T, L228W, R233K/Q, R234W, V269I/M, N272C/G, and K278A numbered according to SEQ ID NO: 4, and
(D') L208C substitution and one or more substitutions selected from the group consisting of L16F, G22C, L50I/V, K123I, R126E, K188Q, L190F, D191A, M212L, V220H, L223V, A224Q/Y, F225T, L228W, R233K, F234W, S256N, V260A/C/L, V269I, N272C/G, K278A, and R286M numbered according to SEQ ID NO: 4.

5. A polynucleotide encoding the lipase variant according to any one of claims 1 to 4.

6. A vector or DNA fragment comprising the polynucleotide according to claim 5.

7. A transformed cell comprising the vector or DNA fragment according to claim 6.

8. The transformed cell according to claim 7, wherein the transformed cell is a microorganism.

9. A cleansing composition comprising the lipase variant according to any one of claims 1 to 4.

10. A method for improving stability of a lipase, comprising the step of substituting one or more amino acid residues at positions selected from the group consisting of positions corresponding to positions L16, G22, Y29, Y43, A45, T46, L50, S120, K123, R126, T134, L142, A145, G156, K174, A181, Q186, K188, L190, D191, L208, H209, M212, L215, V220, A221, L223, A224, F225, L228, R233, F234, S256, V260, L266, V269, N272, T274, K278, and R286 numbered according to SEQ ID NO: 4, wherein the lipase or a lipase after amino acid residue substitution of the lipase has a sequence identity of at least 80% with the amino acid sequence of SEQ ID NO: 4 or 6.
